# EUROPEAN PATENT APPLICATION

(11) **EP 4 190 801 A1**
(43) Date of publication of application: **07.06.2023**
(21) Application number: 21850666.5
(22) Date of filing: 29.07.2021
(51) Int. Cl.: C07K 14/00, C07K 16/00, C12N 5/07, C12N 15/11, G01N 33/15, G01N 33/50, G01N 33/58, G01N 27/62

(54) **METHOD FOR MEASURING PHARMACOKINETICS OF DRUG LABELED WITH NON-RADIOACTIVE SUBSTANCE**

(30) Priority: 29.07.2020 JP 2020128088
(71) Applicant: CHUGAI SEIYAKU KABUSHIKI KAISHA, Tokyo 115-8543 (JP)
(72) Inventor: NAMBU, Takeru, Gotemba-shi, Shizuoka 412-8513 (JP); OZEKI, Kazuhisa, Gotemba-shi, Shizuoka 412-8513 (JP); TAKANO, Yoko, Gotemba-shi, Shizuoka 412-8513 (JP); WATANABE, Miho, Gotemba-shi, Shizuoka 412-8513 (JP)
(74) Representative: Vossius & Partner Patentanwälte Rechtsanwälte mbB
(86) International application number: PCT/JP2021/028099
(87) International publication number: WO 2022/025184

(57) **Abstract**

The present invention provides a method for labeling an agent, the method comprising: (i) a step of chelating a non-radioactive substance by a chelating agent having a reactive group, and (ii) a step of binding the chelating agent that chelated the non-radioactive substance in step (i) to the agent via the reactive group, the method being for use in the evaluation of pharmacokinetics, and the like.

## Description

### TECHNICAL FIELD

The present invention relates to a method for labeling an agent with a non-radioactive substance, a method for measuring the pharmacokinetics of an agent labeled with a non-radioactive substance, a method for screening agents with a desired pharmacokinetics, and the like.

### BACKGROUND ART

Conventionally, in the process of developing a candidate drug substance (e.g., a high-molecular-weight protein preparation typified by antibodies), a method of administering the candidate drug substance labeled with a radioisotope (RI) to laboratory animals such as mice and monkeys and measuring the radioactivity in the tissues or blood collected after a certain period of time to evaluate the pharmacokinetics of the candidate drug substance is known as a general method for evaluating the pharmacokinetics such as the organ distribution and changes in blood concentration of the candidate substance (Non Patent Literature 1).

The advantages of this method are that it directly measures the radioactivity derived from the RI, thus allowing absolute quantification of the candidate drug substance, and that it is little affected by the matrix (i.e., substances other than those to be measured) contained in the tissue or blood. With general quantitative methods, it is necessary to perform an operation of separating and recovering the object to be measured from the matrix using an organic solvent or a column. In such cases, the analysis may be difficult due to problems such as the inability to sufficiently recover the object. With a method using RI, such problems need little consideration.

On the other hand, conducting experiments using RI is limited to specially licensed and dedicated facilities, from the viewpoint of radiation exposure and the need to contain the radioactive substances. Therefore, experiments using RI are not possible without those who have special knowledge of the use of RI using equipment and animal breeding facilities located in a RI facility. There are also various disadvantages associated with experiments using RI, such as the operational complexity of having to conduct experiments while taking special protective measures to prevent radiation exposure, the need for measures to prevent contamination by radioactive substances, and the problem of disposal of waste contaminated by the radioactive substances.

Furthermore, the amount of each nuclide used is limited to the extent permitted by the regulatory authority. In the early stages of drug development, it is necessary to evaluate a large number of candidate substances, but due to such usage limitations, it is not possible to evaluate all candidate substances using RI. In order to perform pharmacokinetic evaluation using RI, candidate substances must be narrowed down beforehand.

To avoid the disadvantages of experiments using RI as mentioned above, methods for evaluating pharmacokinetics without using RI have been used. For example, methods using fluorescent-labeled candidate substances are known as such methods.

Methods using LC-MS and immunoassay are also known as methods for evaluating candidate substances without labeling them. The blood concentration of a candidate substance is generally measured by immunoassay. In immunoassays, enzyme-labeled antibodies or fluorescent-labeled antibodies are commonly used, and the blood concentration of the candidate substance is quantified by measuring the chemiluminescence or fluorescence. Immunoassay methods using antibodies labeled with metals such as europium, samarium, terbium, and dysprosium, and gold colloids have also been reported (Patent Literatures 1 and 2, Non Patent Literatures 2 and 3). In these methods, inductively coupled plasma-mass spectrometry (ICP-MS) is used to detect the metals, and because of its high detection sensitivity, it is also employed in clinical testing.

ICP-MS is a technique that allows ultratrace analysis (ppt level) of many inorganic elements, mainly metallic elements. This technique has many advantages, such as the ability to analyze multiple elements simultaneously, to measure isotope ratio, and to conduct highly accurate and rapid analysis, and is widely applied in the industry of materials such as semiconductors, and in fields such as geology and the environment (Patent Literature 3). ICP-MS is also used as a method for quantitative determination of individual elemental impurities in drugs. There are also reports of cases in which metal colloids were administered to living organisms and the *in vivo* kinetics of the metals were evaluated with high sensitivity by ICP-MS.

### CITATION LIST

### PATENT LITERATURE

[Patent Literature 1] US 2004/0072250 A1
[Patent Literature 2] US 7767407 B2
[Patent Literature 3] Japanese Patent No. 4585069

### NON PATENT LITERATURE

[Non Patent Literature 1] Glassman et al., The Journal of Clinical Pharmacology Vol 55 No.53 (2015), 529-538
[Non Patent Literature 2] J. Anal. At. Spectrom., 2001, 16, 1393-1396
[Non Patent Literature 3] Mass Spec Rev 29:326-348, 2010

### SUMMARY OF INVENTION

### TECHNICAL PROBLEM

The method for evaluating pharmacokinetics using RI has various disadvantages, as described above. In particular, it has been difficult to conduct screenings based on pharmacokinetics in the early stages of drug development due to the existence of limits on RI usage. Therefore, there is a significant need for a method to evaluate pharmacokinetics without using RI. However, the methods known in the prior art were no substitute for methods using RI. For example, when fluorescent labeling is used, it is affected by the matrix contained in the tissue or blood, and therefore requires an operation to extract the object to be measured from the biological sample. The efficiency of extraction cannot be 100%, resulting in a partial loss in the object to be measured, and thus making it difficult to perform an analysis as accurately as the methods using RI.

On the other hand, if the pharmacokinetics of the object to be measured can be evaluated by labeling the object to be measured with a non-radioactive substance and then quantifying the non-radioactive substance by ICP-MS or the like, there is no need to perform the extraction operation as described above. However, so far, there have been no examples of evaluating the pharmacokinetics of an object to be measured by labeling the object to be measured with a non-radioactive substance, administering it to an animal, and then following the kinetics of the non-radioactive substance. Reasons for this include the difficulties in preparing labeling substances suitable for pharmacokinetic evaluation, such as the difficulty in removing the non-radioactive substances that have not been incorporated into the object to be measured.

An object of the present invention is to provide: 1) a method for labeling an agent serving as a candidate drug substance, such as an antibody, with a non-radioactive substance, 2) a method for measuring the pharmacokinetics of the agent labeled with a non-radioactive substance by this method, 3) a method for screening agents with a desired pharmacokinetics, and the like.

### SOLUTION TO PROBLEM

As a result of extensive research, the present inventors have found a novel method enabling the labeling of an agent with a non-radioactive substance in a way suitable for pharmacokinetic evaluation. Conventionally, when labeling an agent with a RI, a chelating agent was bound to the agent and then the RI was chelated. The present inventors have found that when this labeling method is applied to non-radioactive substances, evaluating the pharmacokinetics of the agent based on the content of non-radioactive substance in the organism did not yield accurate results. However, unexpectedly, the pharmacokinetics of the agent could be accurately evaluated when the non-radioactive substance was chelated by a chelating agent and then the chelating agent was bound to the agent. Based on these findings, the present inventors conducted further research to complete the present invention.

That is, the present invention provides the following inventions.
[1] A method for labeling an agent, the method comprising:
   (i) a step of chelating a non-radioactive substance by a chelating agent having a reactive group, and
   (ii) a step of binding the chelating agent that chelated the non-radioactive substance in step (i) to the agent via the reactive group,
      the method being for use in the evaluation of pharmacokinetics.
[2] The method according to [1], wherein the non-radioactive substance is a metal.
[3] The method according to [2], wherein the non-radioactive substance is lithium, beryllium, sodium, magnesium, aluminum, potassium, calcium, scandium, titanium, vanadium, chromium, manganese, iron, cobalt, nickel, copper, zinc, gallium, rubidium, strontium, yttrium, zirconium, niobium, molybdenum, ruthenium, rhodium, palladium, silver, cadmium, indium, tin, cesium, barium, lanthanum, cerium, praseodymium, neodymium, samarium, europium, gadolinium, terbium, dysprosium, holmium, erbium, thulium, ytterbium, lutetium, hafnium, tantalum, tungsten, rhenium, osmium, iridium, platinum, gold, mercury, thallium, lead, bismuth, or thorium.
[4] The method according to [3], wherein the non-radioactive substance is indium or europium.
[5] The method according to [4], wherein the non-radioactive substance is indium.
[6] The method according to any one of [1] to [5], wherein the chelating agent having a reactive group is a chelating agent having an amino group or a carboxyl group as a coordinating group.
[7] The method according to [6], wherein the chelating agent having a reactive group is a chelating agent having a reactive group introduced into any compound selected from DOTA, EDTA, HEDTA, DHEDDA, 1,3-PDTA, DTPA, TTHA, NTA, gluconic acid, HIMDA, ASDA, NTMP, HEDP, tetrasodium 3-hydroxy-2,2'-iminodisuccinate, and porphyrin.
[8] The method according to [7], wherein the chelating agent having a reactive group is DOTA with a reactive group introduced.
[9] The method according to any one of [1] to [8], wherein the agent is an antibody, a peptide compound, a cell, or a nucleic acid.
[10] The method according to any one of [1] to [9], wherein the reactive group is a group capable of forming a chemical bond by reacting with an amino group or a thiol group.
[11] The method according to [10], wherein the reactive group is an NHS ester, an ITC group, or a maleimide group.
[12] The method according to [11], wherein the reactive group is an ITC group.
[13] A method for measuring the pharmacokinetics of an agent, the method comprising:
   (i) a step of labeling the agent with a non-radioactive substance by the method according to any one of [1] to [12],
   (ii) a step of administering the agent labeled in step (i) to a non-human animal, and
   (iii) a step of collecting a biological sample from the non-human animal after administration of the agent in step (ii) and measuring the content of the non-radioactive substance in the biological sample.
[14] The method according to [13], wherein in step (i), two or more agents are labeled with different non-radioactive substances,
   and in step (ii), the two or more agents labeled in step (i) are administered to a single non-human animal.
[15] The method according to [13] or [14], wherein the content of the non-radioactive substance is measured by spectrometry or mass spectrometry.
[16] The method according to [15], wherein the content of the non-radioactive substance is measured by mass spectrometry.
[17] The method according to [16], wherein the mass spectrometry is plasma ionization mass spectrometry, electron ionization (EI) mass spectrometry, chemical ionization (CI) mass spectrometry, atmospheric pressure chemical ionization (APCI) mass spectrometry, electrospray ionization (ESI) mass spectrometry, matrix-assisted laser desorption/ionization (MALDI) mass spectrometry, laser desorption mass spectrometry (LDMS), secondary ion mass spectrometry (SIMS), spark source mass spectrometry (SSMS), or thermal ionization mass spectrometry (TIMS).
[18] The method according to [17], wherein the mass spectrometry is plasma ionization mass spectrometry.
[19] The method according to [18], wherein the mass spectrometry is inductively coupled plasma (ICP) mass spectrometry, microwave-induced plasma (MIP) mass spectrometry, or glow discharge (GD) mass spectrometry.
[20] The method according to any one of [13] to [19], wherein the non-human animal is a laboratory animal.
[21] The method according to [20], wherein the non-human animal is a monkey, miniature pig, rat, mouse, rabbit, dog, or guinea pig.
[22] The method according to [21], wherein the non-human animal is a mouse.
[23] The method according to [22], wherein the mouse is a cancer-bearing mouse, a pathological model mouse, or a transgenic mouse.
[24] The method according to any one of [13] to [23], wherein the administration of the agent in step (ii) is intravenous administration (i.v.), subcutaneous administration (s.c.), peroral administration (p.o.), intraperitoneal administration (i.p.), intramuscular administration (i.m.), intratumoral administration (i.t.), transpulmonary administration, or intranasal administration.
[25] The method according to any one of [13] to [24], wherein the pharmacokinetics is organ distribution, and
   the biological sample is an organ,
   the method further comprising:
   (iv) a step of determining the distribution of the agent in the organ based on the content of the non-radioactive substance measured in step (iii).
[26] The method according to [25], further comprising:
   (v) a step of quantifying the pharmacokinetics based on the distribution determined in step (iv).
[27] The method according to [25] or [26], wherein the organ is at least one selected from the group consisting of the blood, plasma, testes, ileum, large intestine, jejunum, stomach, liver, lungs, kidneys, spleen, heart, thigh muscles, and skin.
[28] The method according to any one of [13] to [24], wherein the pharmacokinetics is the changes in blood concentration,
   the biological sample is blood, plasma, or serum, and
   step (iii) is a step of collecting the biological sample from the non-human animal twice or more over time after administration of the agent in step (ii) and measuring the content of the non-radioactive substance in the biological sample,
   the method further comprising:
   (iv) a step of determining the changes in blood concentration of the agent based on the content of the non-radioactive substance measured in step (iii).
[29] The method according to [28], further comprising:
   (v) a step of quantifying the pharmacokinetics based on the changes in blood concentration determined in step (iv).
[30] A method for screening agents having a desired pharmacokinetics, the method comprising:
   (i) a step of measuring the pharmacokinetics of two or more candidate agents by the method according to any one of [13] to [29], and
   (ii) a step of comparing the pharmacokinetics of the candidate agents measured in step (i) and selecting an agent exhibiting the desired pharmacokinetics.
[31] A method for producing a composition for pharmacokinetic evaluation comprising an agent labeled with a non-radioactive substance, the method comprising labeling the agent with a non-radioactive substance by the method according to any one of [1] to [30].
[32] A composition for pharmacokinetic evaluation, produced by the method according to [31].

In addition, the present invention provides the following inventions.
[A1] A composition for pharmacokinetic evaluation,
   the composition comprising an agent to which a chelating agent is bound,
   wherein a non-radioactive substance is chelated to the chelating agent.
[A2] The composition according to [A1], wherein the non-radioactive substance is a metal.
[A3] The composition according to [A2], wherein the non-radioactive substance is lithium, beryllium, sodium, magnesium, aluminum, potassium, calcium, scandium, titanium, vanadium, chromium, manganese, iron, cobalt, nickel, copper, zinc, gallium, rubidium, strontium, yttrium, zirconium, niobium, molybdenum, ruthenium, rhodium, palladium, silver, cadmium, indium, tin, cesium, barium, lanthanum, cerium, praseodymium, neodymium, samarium, europium, gadolinium, terbium, dysprosium, holmium, erbium, thulium, ytterbium, lutetium, hafnium, tantalum, tungsten, rhenium, osmium, iridium, platinum, gold, mercury, thallium, lead, bismuth, or thorium.
[A4] The composition according to [A3], wherein the non-radioactive substance is indium or europium.
[A5] The composition according to [A4], wherein the non-radioactive substance is indium.
[A6] The composition according to any one of [A1] to [A5], wherein the chelating agent is a chelating agent having an amino group or a carboxyl group as a coordinating group.
[A7] The composition according to [A6], wherein the chelating agent is any compound selected from DOTA, EDTA, HEDTA, DHEDDA, 1,3-PDTA, DTPA, TTHA, NTA, gluconic acid, HIMDA, ASDA, NTMP, HEDP, tetrasodium 3-hydroxy-2,2'-iminodisuccinate, and porphyrin.
[A8] The composition according to [A7], wherein the chelating agent is DOTA.
[A9] The composition according to any one of [A1] to [A8], wherein the agent is an antibody, a peptide compound, a cell, or a nucleic acid.
[A10] A labeled agent according to any one of [A1] to [A9], wherein the chelating agent is bound to the agent by a chemical bond with an amino group or a thiol group of the agent.

In addition, the present invention provides the following inventions.
[B1] A method for measuring the pharmacokinetics of an agent, the method comprising:
   (i) a step of administering an agent labeled with a non-radioactive substance to a non-human animal, and
   (ii) a step of collecting a biological sample from the non-human animal after administration of the agent in step (i) and measuring the content of the non-radioactive substance in the biological sample.
[B2] The method according to [B1], wherein in step (i), the two or more agents labeled with different non-radioactive substances are administered to a single non-human animal.
[B3] The method according to [B1] or [B2], wherein the content of the non-radioactive substance is measured by spectrometry or mass spectrometry.
[B4] The method according to [B3], wherein the content of the non-radioactive substance is measured by mass spectrometry.
[B5] The method according to [B4], wherein the mass spectrometry is plasma ionization mass spectrometry, electron ionization (EI) mass spectrometry, chemical ionization (CI) mass spectrometry, atmospheric pressure chemical ionization (APCI) mass spectrometry, electrospray ionization (ESI) mass spectrometry, matrix-assisted laser desorption/ionization (MALDI) mass spectrometry, laser desorption mass spectrometry (LDMS), secondary ion mass spectrometry (SIMS), spark source mass spectrometry (SSMS), or thermal ionization mass spectrometry (TIMS).
[B6] The method according to [B5], wherein the mass spectrometry is plasma ionization mass spectrometry.
[B7] The method according to [B6], wherein the mass spectrometry is inductively coupled plasma (ICP) mass spectrometry, microwave-induced plasma (MIP) mass spectrometry, or glow discharge (GD) mass spectrometry.
[B8] The method according to any one of [B1] to [B7], wherein the non-human animal is a laboratory animal.
[B9] The method according to [B8], wherein the non-human animal is a monkey, miniature pig, rat, mouse, rabbit, dog, or guinea pig.
[B10] The method according to [B9], wherein the non-human animal is a mouse.
[B11] The method according to [B10], wherein the mouse is a cancer-bearing mouse, a pathological model mouse, or a transgenic mouse.
[B12] The method according to any one of [B1] to [B 11], wherein the administration of the agent in step (i) is intravenous administration (i.v.), subcutaneous administration (s.c.), peroral administration (p.o.), intraperitoneal administration (i.p.), intramuscular administration (i.m.), intratumoral administration (i.t.), transpulmonary administration, or intranasal administration.
[B13] The method according to any one of [B1] to [B12], wherein the pharmacokinetics is organ distribution, and
   the biological sample is an organ,
   the method further comprising:
   (iii) a step of determining the distribution of the agent in the organ based on the content of the non-radioactive substance measured in step (ii).
[B14] The method according to [B13], further comprising:
   (iv) a step of quantifying the pharmacokinetics based on the distribution determined in step (iii).
[B15] The method according to [B13] or [B14], wherein the organ is at least one selected from the group consisting of the blood, plasma, testes, ileum, large intestine, jejunum, stomach, liver, lungs, kidneys, spleen, heart, thigh muscles, and skin.
[B16] The method according to any one of [B1] to [B 12], wherein the pharmacokinetics is the changes in blood concentration,
   the biological sample is blood, plasma, or serum, and
   step (ii) is a step of collecting the biological sample from the non-human animal twice or more over time after administration of the agent in step (i) and measuring the content of the non-radioactive substance in the biological sample,
   the method further comprising:
   (iii) a step of determining the changes in blood concentration of the agent based on the content of the non-radioactive substance measured in step (ii).
[B17] The method according to [B16], further comprising:
   (iv) a step of quantifying the pharmacokinetics based on the changes in blood concentration determined in step (iii).
[B18] A method for screening agents having a desired pharmacokinetics, the method comprising:
   (i) a step of measuring the pharmacokinetics of two or more candidate agents by the method according to any one of [B1] to [B 17], and
   (ii) a step of comparing the pharmacokinetics of the candidate agents measured in step (i) and selecting an agent exhibiting the desired pharmacokinetics.

### ADVANTAGEOUS EFFECTS OF INVENTION

In the method of the present invention, there is no need to label the agent to be measured with RI. Therefore, in the method of the present invention, there are no limitations on the number of measurable specimens which arise from the limitations on RI usage or limitations on the test site, as in the methods using agents labeled with RI, and there is also no need to secure researchers with expertise in RI. Therefore, according to the present invention, it is possible to evaluate the pharmacokinetics of a large number of candidate substances easily and with high accuracy in the early stages of drug development.

Furthermore, according to the present invention, it is possible to administer a plurality of candidate substances having different types of non-radioactive substances used for labeling to a single animal and to measure each separately. Therefore, the present invention can contribute to the reduction of the number of animals used in pharmacokinetic studies.

The present invention can also contribute to the reduction of resources required for installing and maintaining dedicated facilities for containing radioactive substances, the reduction of radioactive waste, and the elimination of the risk of exposure to radiation for those involved in the measurements.

Furthermore, the present invention can contribute to the development of more pharmacologically effective drugs by providing a method for efficiently screening agents with a desired pharmacokinetics.

### BRIEF DESCRIPTION OF DRAWINGS

Figure 1 shows the results of measuring the plasma concentration of In-DOTA-labeled cetuximab prepared in Example 1 in mice using Ligand-Binding Assay (LBA) or ICP-MS. Each point indicates the mean of one group (n=3) and the error bars indicate S.D.
Figure 2 shows the concentration of In-DOTA-labeled cetuximab prepared in Example 2 in mouse tissues 24 hours after administration. Each point indicates the mean of one group (n=3) and the error bars indicate S.D.
Figure 3 shows the results of measuring the plasma concentration of In-labeled DTPA-cetuximab prepared in Comparative Example 1 in mice using LBA or ICP-MS. Each point indicates the mean of one group (n=3) and the error bars indicate S.D.
Figure 4 shows the results of measuring the plasma concentration of iodine-labeled denosumab prepared in Example 3, which was labeled by the Iodogen method, in mice using LBA or ICP-MS. Each point indicates the mean of one group (n=3) and the error bars indicate S.D.
Figure 5 shows the concentration of iodine-labeled denosumab and indium-DOTA-labeled denosumab prepared in Example 4 in mouse tissues on day 7 after administration. Each bar indicates the mean of one group (n=3) and the error bars indicate S.D.
Figure 6 shows the results of dose normalizing the measured plasma concentrations of iodine-labeled denosumab and indium-DOTA-labeled denosumab prepared in Example 4, in mice using ICP-MS, and of the measured plasma concentrations of denosumab in mice using LBA. Each point indicates the mean of one group (n=3) and the error bars indicate S.D.
Figure 7 shows the concentrations of the four metal-labeled antibodies prepared in Example 5 in mouse tissues 7 days after administration. Each bar indicates the mean of one group (n=3) and the error bars indicate S.D.
Figure 8 shows the results of dose normalizing the measured plasma concentrations of indium-DOTA-labeled cetuximab, terbium-DOTA-labeled cetuximab, yttrium-DOTA-labeled cetuximab, and holmium-DOTA-labeled cetuximab prepared in Example 5, in mice using ICP-MS, and of the measured plasma concentrations of cetuximab in mice using LBA. Each point indicates the mean of one group (n=3) and the error bars indicate S.D.
Figure 9 shows the concentration of indium-DOTA-labeled antibodies prepared in Example 6 in monkey tissues 7 days after administration. Each bar indicates the mean of one group (n=3) and the error bars indicate S.D.
Figure 10 shows the results of measuring the plasma concentration of indium-DOTA-labeled antibodies prepared in Example 6 in monkey using LBA or ICP-MS. Each point indicates the mean of one group (n=3) and the error bars indicate S.D.

### DESCRIPTION OF EMBODIMENTS

Hereinafter, the embodiments of the present invention are described in detail. However, the present invention is not limited thereto, and can be carried out with various modified aspects within the described scope. Unless otherwise noted herein, "A to B" representing a numerical range means "A or more (including A and greater than A) and B or less (including B and less than B)." In addition, all references cited herein are incorporated herein by reference.

### I. Method for Labeling Agent

The first aspect of the present invention relates to a method for labeling an agent, the method comprising:
(i) a step of chelating a non-radioactive substance by a chelating agent having a reactive group, and
(ii) a step of binding the chelating agent that chelated the non-radioactive substance in step (i) to the agent via the reactive group,
   the method being for use in the evaluation of pharmacokinetics (hereinafter also referred to as the Method I of the present invention).

In the present invention, the "agent" is not particularly limited as long as chelating agents can bind to it via a reactive group and it can be the object of pharmacokinetic evaluation. The "agent" includes drugs and candidates thereof, and examples thereof include proteins such as peptide compounds, nucleic acids, and antibodies, and cells. In agents such as molecules and cells that are sufficiently large compared to the chelating agent, the binding of the chelating agent is unlikely to produce an effect on their pharmacokinetics. From this viewpoint, if the agent of the present invention is a molecule such as a peptide compound, a nucleic acid or a protein, its molecular weight is preferably 500 or more. In a preferred aspect, the agent is a peptide compound, a nucleic acid, an antibody, or a cell, and is particularly preferably an antibody.

In the present invention, "peptide compound" refers to a compound formed by amide or ester bonding of amino acids. In the present invention, the "amino acids" constituting the peptide compound can be "natural amino acids" or "amino acid analogs." The "amino acid," "natural amino acid," and "amino acid analog" are sometimes referred to as "amino acid residue," "natural amino acid residue," and "amino acid analog residue," respectively.

The amino acids constituting the peptide compound can be α-amino acids, β-amino acids, or γ-amino acids. In the case of α-amino acids, they may be either L-amino acids or D-amino acids, or α,α-dialkylamino acids. The selection of an amino acid side chain is not particularly limited, and the side chain is freely selected from, in addition to a hydrogen atom, an alkyl group, an alkenyl group, an alkynyl group, an aryl group, a heteroaryl group, an aralkyl group, a cycloalkyl group, and the like. A substituent may be added to each of the groups, and these substituents are freely selected, for example, from arbitrary functional groups including a N atom, an O atom, a S atom, a B atom, a Si atom, and a P atom (i.e., an optionally substituted alkyl group, alkenyl group, alkynyl group, aryl group, heteroaryl group, aralkyl group, cycloalkyl group, and the like).

In the present invention, natural amino acids refer to α-aminocarboxylic acids (α-amino acids, which are the 20 amino acids contained in naturally occurring proteins, specifically Gly, Ala, Ser, Thr, Val, Leu, Ile, Phe, Tyr, Trp, His, Glu, Asp, Gln, Asn, Cys, Met, Lys, Arg, and Pro).

"Amino acid analogs" include unnatural amino acids (e.g., unnatural α-amino acids, β-amino acids, and γ-amino acids). In the case of α-amino acids, they may be D-amino acids or α,α-dialkylamino acids. In the case of β- or γ-amino acids, any conformation is acceptable, as with α-amino acids. The side chain (main chain methylene) of an amino acid analog is not particularly limited, and can have, for example, in addition to a hydrogen atom, an alkyl, an alkenyl, an alkynyl, an aryl, a heteroaryl, an aralkyl, or a cycloalkyl. They may each have one or more substituent, and these substituents can be selected, for example, from arbitrary functional groups including a halogen atom, a N atom, an O atom, a S atom, a B atom, a Si atom, and a P atom. The amino group of the main chain of the amino acid analog may be unsubstituted (NH₂ group), or substituted (i.e., NHR group: R represents an alkyl, an alkenyl, an alkynyl, an aryl, a heteroaryl, an aralkyl, or a cycloalkyl, which may have substituents, or a carbon chain bound to the N atom and a carbon atom at position α may form a ring, like proline. The substituents are the same as those of the side chain, and examples thereof include a halogen, oxy, and hydroxy). "Amino acid analogs" also include hydroxycarboxylic acids, in which the amino group of an "amino acid" is replaced by a hydroxyl group. The hydroxycarboxylic acid may have various substituents, like the other amino acid analogs. The conformation of the hydroxycarboxylic acid may correspond to either the L- or D-form of the amino acid. The side chain is not particularly limited, but is selected from, for example, an optionally substituted alkyl, alkenyl, alkynyl, aryl, heteroaryl, aralkyl, and cycloalkyl. The "amino acid analogs" known to those skilled in the art can be utilized. See, for example, WO2017/150732.

The molecular form of the peptide compound can be linear, cyclic, or cyclic with a linear portion.

The number of amide or ester bonds (the number and length of amino acid residues) in the peptide compound is not particularly limited, and can be any number corresponding to a molecular weight of 500 or more and less than the molecular weight of a protein such as an antibody, for example. The number of amino acids constituting the peptide compound can be, for example, 50 residues or less, preferably 30 residues or less, and more preferably 13 residues or less, including both cyclic and linear portions. In addition, the number of amino acids constituting the peptide compound can be, for example, 5 residues or more, and to obtain high metabolic stability, more preferably 9 residues or more. If the peptide compound has a cyclic portion, the number of amino acids constituting the cyclic portion is preferably 5 to 12 residues, more preferably 5 to 11 residues, still more preferably 7 to 11 residues, and particularly preferably 9 to 11 residues. The number of amino acids in the linear portion is preferably 0 to 8 residues, and more preferably 0 to 3 residues.

In the present invention, "nucleic acid" refers to DNA, RNA, and the analogs thereof, and may be a natural nucleic acid or a synthetic nucleic acid. Examples of analogs include artificial nucleic acids such as PNA and LNA. The nucleic acid may be single-stranded or double-stranded. The nucleic acid may also be modified. Examples of modified nucleic acids include those chemically modified at the internucleoside linkages, bases and/or sugars, and those having a modified group at the 5' end and/or 3' end. Modifications of the internucleoside linkages include changes to any of the phosphodiester linkages, phosphorothioate linkages, phosphorodithioate linkages, methylphosphonate linkages, phosphoramidate linkages, non-phosphate linkages, and methylphosphonothioate linkages, or a combination thereof. Examples of base modifications include changes to 5-propynyluracil, 2-aminoadenine, and the like. Sugar modifications include changes to 2'-fluororibose, 2'-O-methyl ribose, and the like.

Nucleic acids are sometimes referred to as siRNA, antisense RNA, miRNA, shRNA, ribozymes, or aptamers, depending on their function or use. Nucleic acids also include oligonucleotides having adjuvant effects, such as CpG oligonucleotides that act on Toll-like receptor 9 (TLR9) to activate innate immunity.

The length of the nucleic acid is not particularly limited as long as the chelating agent can bind via the reactive group, and can range, for example, from 4 to 100 bases long, 10 to 50 bases long, 10 to 40 bases long, or 10 to 30 bases long.

The "protein" as an agent in the present invention is a long-chain polymer of amino acids linked via peptide bonds, and can also include peptide compounds. The protein may be naturally occurring, or not naturally occurring, such as recombinant proteins. Examples of proteins include cytokines, bioactive peptides, biological enzymes, antibodies, or mutants thereof.

In the present invention, the term "antibody" refers to immunoglobulin that is natural or produced through partial or complete synthesis, or its antigen-binding fragment. An antibody can be isolated from a natural resource such as plasma and serum in which the antibody is naturally present and a culture supernatant of hybridoma cells producing the antibody, and can be partially or completely synthesized by using a technique of gene recombination or the like. Preferred examples of the antibody include isotypes of immunoglobulin (i.e., IgG, IgA, IgD, IgE, and IgM) and subclasses of these isotypes. Known as human immunoglobulin are nine subclasses: IgG1, IgG2, IgG3, IgG4, IgA1, IgA2, IgD, IgE, and IgM. In a preferred aspect, the antibody as the agent in the Method I of the present invention can be IgG.

The antibody may be either a polyclonal antibody or a monoclonal antibody. In the present invention, it may also be a recombinant antibody that has been artificially altered for the purpose of reducing heteroantigenicity and the like, such as chimeric antibodies and humanized antibodies. The antibody may also be a bispecific antibody or a multispecific antibody.

The antibody may be a fragment of an antibody as long as it contains an "antigen binding domain," i.e., an antigen binding fragment. Examples of fragments of antibodies include, but are not limited to: Fv, Fab, Fab', Fab'-SH, F(ab')2; diabody; linear antibodies; single-chain antibody molecules (e.g., scFv and VHH); and multispecific antibodies formed from antibody fragments. The "antigen binding domain" in the antibody is any domain that binds to the antigen of interest, and is, for example, the variable region of the heavy chain or light chain of the antibody.

In addition, in one embodiment of the present invention, the antibody can be a polypeptide that contains an antigen binding domain and a carrying moiety having an inhibiting domain which inhibits the antigen binding activity of the antigen binding domain, and that has a longer half-life than the antigen binding domain present alone (see WO2019/107380). In the polypeptide according to this embodiment, by releasing the antigen binding domain from the polypeptide, the antigen binding activity is higher than before the release. In other words, when the antigen binding domain is not released from the polypeptide, its antigen binding activity is inhibited by the inhibiting domain. Whether the antigen binding activity of the antigen binding domain is inhibited by the inhibiting domain is confirmed by a method such as FACS (fluorescence activated cell sorting), ELISA (enzyme-linked immunosorbent assay), ECL (electrogenerated chemiluminescence), a SPR (surface plasmon resonance) method (Biacore), BLI (biolayer interferometry) (Octet). In the polypeptide according to this embodiment, the antigen binding activity when the antigen binding domain is released from the polypeptide is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the binding activity when the antigen binding domain is not released from the polypeptide. In the specific polypeptide according to this embodiment, the binding of the antigen binding domain before the release to the antigen is not seen when the antigen binding activity of the antigen binding domain is measured by one method selected from among the methods described above.

In the polypeptide according to this embodiment, the carrying moiety in the polypeptide may also be linked to the antigen binding domain via a cleavage site. In this case, the cleavage of the cleavage site allows the antigen binding domain to be released from the polypeptide, and the antigen binding activity in such aspect can thus be compared by comparing the antigen binding activity before and after cleavage of the polypeptide. Specifically, the antigen binding activity measured using the cleaved polypeptide is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the antigen binding activity measured using the uncleaved polypeptide. In the specific polypeptide according to this embodiment, the binding of the antigen binding domain of the uncleaved polypeptide to the antigen is not seen when the antigen binding activity is measured by one method selected from among the methods described above.

Furthermore, in the polypeptide according to this embodiment, the cleavage site can be cleaved by a protease. In this case, the antigen binding activity can be compared by comparing the antigen binding activity between before and after the protease treatment of the polypeptide. Specifically, the antigen binding activity measured using the polypeptide after the protease treatment is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1000 times, 2000 times, or 3000 times the antigen binding activity measured using the polypeptide without the protease treatment. In the specific polypeptide according to this embodiment, the binding of the antigen binding domain of the protease-untreated polypeptide to the antigen is not seen when the antigen binding activity is measured by one method selected from among the methods described above.

The methods for preparing these antibodies are known to those skilled in the art (see, for example, WO2019/107380 and WO2013/081143).

The antibody also includes immunoconjugates, such as antibodies conjugated to one or more cytotoxic agents (e.g., chemotherapeutic agents or chemotherapeutic drugs, growth inhibitors, or toxins (e.g., protein toxins of bacterial, fungal, plant or animal origin, enzymatically active toxins, or fragments thereof)).

In one aspect, the immunoconjugate is an antibody-drug conjugate (ADC) in which the antibody is conjugated to one or more drugs, including, but not limited to: maytansinoids (see U.S. Patent Nos. 5,208,020 and 5,416,064, and European Patent No. 0,425,235 B1); auristatins such as monomethyl auristatin drug moieties DE and DF (MMAE and MMAF) (see U.S. Patent Nos. 5,635,483, 5,780,588 and 7,498,298); dolastatins; calicheamicin or derivatives thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); anthracyclines such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; taxanes such as docetaxel, paclitaxel, larotaxel, tesetaxel, and ortataxel; trichothecenes; and CC1065.

In another aspect, the immunoconjugate includes antibodies conjugated to enzymatically active toxins or fragments thereof, including, but not limited to: diphtheria-A chain, non-binding active fragments of diphtheria toxins, exotoxin A chain (from *Pseudomonas aeruginosa*)*,* ricin A chain, abrin A chain, modeccin A chain, alphasarcin, *Aleurites fordii* proteins, dianthin proteins, *Phytolacca americana* proteins (PAPI, PAPII and PAP-S), momordica charantia inhibitor, curcin, crotin, saponaria officinalis inhibitor, gelonin, mitogellin, restrictocin, phenomycin, enomycin, and trichothecenes.

The conjugates of antibodies and cytotoxic agents can be prepared using various bifunctional protein linking agents. Examples thereof include N-succinimidyl-3-(2-pyridyldithio)propionate (SPDP), succinimidyl-4-(N-maleimidomethyl)cyclohexane-1-carboxylate (SMCC), iminothiolane (IT), bifunctional derivatives of imido esters (e.g., dimethyl adipimidate HCl), active esters (e.g., disuccinimidyl suberate), aldehydes (e.g., glutaraldehyde), bis-azido compounds (e.g., bis(p-azidobenzoyl)hexanediamine), bisdiazonium derivatives (e.g., bis-(p-diazonium benzoyl)-ethylenediamine), diisocyanates (e.g., toluene 2,6-diisocyanate), and bis-active fluorine compounds (e.g., 1,5-difluoro-2,4-dinitrobenzene). For example, ricin immunotoxins can be prepared as described in Vitetta et al., Science 238:1098 (1987). The immunoconjugate can contain a linker between the antibody and the cytotoxic agent. The linker can be a "cleavable linker" that promotes the release of the cytotoxic drug into the cell. For example, acid-labile linkers, peptidasesensitive linkers, photolabile linkers, dimethyl linkers, or disulfide-containing linkers (Chari et al., Cancer Res. 52: 127-131 (1992); U.S. Patent No. 5,208,020) can be used.

As the immunoconjugates or ADCs of the present specification, the conjugates prepared with cross-linking reagents including but not limited to the commercially available (e.g., from Pierce Biotechnology, Inc., Rockford, IL., U.S.A.) BMPS, EMCS, GMBS, HBVS, LC-SMCC, MBS, MPBH, SBAP, SIA, SlAB, SMCC, SMPB, SMPH, sulfo-EMCS, sulfo-GMBS, sulfo-KMUS, sulfo-MBS, sulfo-SIAB, sulfo-SMCC, sulfo-SMPB, and SVSB (succinimidyl-(4-vinylsulfone)benzoate) are explicitly considered, but not limited thereto.

The "cells" as an agent in the present invention are not particularly limited as long as they can be the object of pharmacokinetic evaluation, but examples thereof include cells used in cell therapies such as stem cell transplantation and adoptive cell immunotherapy. Examples of such cells include various types of stem cells (mesenchymal stem cells, hematopoietic stem cells, ES cells, iPS cells, etc.), chimeric antigen receptor-expressing T cells (CAR-T cells), and T cell receptor-expressing T cells (TCR-T cells).

In the present invention, "pharmacokinetics" (also referred to as PK) refers to the changes in the concentration (amount) of an agent in a living body through a series of processes such as absorption, distribution, metabolism, and excretion in the living body after the agent is administered.

After an agent is administered, the processes of absorption, distribution, metabolism, and excretion proceed in parallel in a living body. As the basic PK parameters to break down and describe these processes, (1) bioavailability (F), (2) volume of distribution (Vd or V), (3) fraction unbound in blood (fuB), (4) clearance (CL), and (5) excretion rate in urine (cumulative amount of drug excreted in urine: Ae) have been established (Biometrics Vol. 36, Special Issue, S 3-S 18 (2015)). Area under the blood concentration-time curve (AUC), maximum blood concentration (Cmax), time to reach maximum blood concentration (Tmax), and the like are known as indicators of bioavailability. Volume of distribution at steady state (Vₛₛ) and the like are known as indicators of volume of distribution. As other PK parameters, blood concentration half-life (t_{1/2}), mean residence time (MRT), and the like are known.

The Method I of the present invention is a method for use in the evaluation of pharmacokinetics. In the present invention, the agent is an object of pharmacokinetic evaluation and is labeled with a non-radioactive substance for pharmacokinetic evaluation. That is, the labeled agent obtained by the method of the present invention is used in the evaluation of pharmacokinetics. Evaluation of pharmacokinetics can be appropriately carried out according to a method known in the art such as the "Guidelines for Nonclinical Pharmacokinetic Studies" (PMSB/ELD Notification No. 496, June 26, 1998), or it can also be carried out according to Method II of the present invention described below.

In the present invention, the "chelating agent" is not particularly limited as long as it can be used in the field of pharmaceuticals, and can be appropriately selected from chelating agents usually used by those skilled in the art. Examples of chelating agents include DOTA (1,4,7,10-tetraazacyclododecane-1,4,7,10-tetraacetic acid), EDTA (ethylenediaminetetraacetic acid), HEDTA (N-(2-hydroxyethyl)ethylenediamine-N,N',N'-triacetic acid), DHEDDA (dihydroxyethylethylenediamine-diacetic acid), 1,3-PDTA (1,3-propanediaminetetraacetic acid), DTPA (diethylenetriaminepentaacetic acid), TTHA (triethylenetetramine-N,N,N',N",N‴,N‴-hexacetic acid), NTA (nitrilotriacetic acid), gluconic acid, HIMDA (hydroxyethyliminodiacetic acid), ASDA (L-aspartic diacetic acid), NTMP (nitrilotrimethylene phosphonic acid), HEDP (1-hydroxyethylidene-1,1-diphosphonic acid), tetrasodium 3-hydroxy-2,2'-iminodisuccinate, phenanthroline, porphyrins, and crown ethers.

In a preferred aspect, the chelating agent having a reactive group is a chelating agent having an amino group or a carboxyl group as a coordinating group, and for example, can be a chelating agent having a reactive group introduced into any compound selected from DOTA, EDTA, HEDTA, DHEDDA, 1,3-PDTA, DTPA, TTHA, NTA, gluconic acid, HIMDA, ASDA, NTMP, HEDP, tetrasodium 3-hydroxy-2,2'-iminodisuccinate, and porphyrins, more preferably DOTA or DTPA with a reactive group introduced into the compound.

The "reactive group" in the chelating agent is a group that can form a covalent bond with the agent, and can be appropriately selected according to the type of agent and the type of reaction used to bind the agent to the chelating agent. The bond between the agent and the chelating agent is preferably not easily cleaved in the living body.

Examples of the reactive groups that can bind to the amino groups (NH₂ groups) in the agent include N-hydroxysuccinimide esters (NHS esters), isothiocyano groups (ITC groups), sulfonic acid chlorides, carboxylic acid chlorides, ethylene oxides, alkyl chlorides, aldehyde groups, and carboxylic acid anhydrides, preferably NHS esters or ITC groups, and more preferably ITC groups.

Examples of the reactive groups that can bind to the thiol groups (SH groups) in the agent include maleimide groups and bromoacetamide groups, preferably maleimide groups.

If the agent is a nucleic acid, for example, a carbodiimide cross-linking agent (such as 1-ethyl-3-(3-dimethylaminopropyl)carbodiimide (EDC) and N',N'-dicyclohexylcarbodiimide (DCC)) or a photoreactive cross-linking agent (such as phenyl azide and psoralen) can be used to bind a chelating agent with a reactive group, chelating a non-radioactive substance, to the agent. If a carbodiimide cross-linking agent is used, the reactive group is an amino group. If a photoreactive cross-linking agent is used, the reactive group is a nucleophilic group or an active hydrogen group. See Bioconjugate Chem., vol. 1, No. 3, 1990, 165-187. The synthesis of a nucleic acid, the agent, after binding a chelating agent with a reactive group, chelating a non-radioactive substance, to a nucleotide constituting the agent is also included in the Method I of the present invention.

If the agent is a nucleic acid, the chelating agent with a reactive group can also be bound to the agent by chelating a non-radioactive substance to a nucleotide bound to the chelating agent (which corresponds to the chelating agent with a reactive group) and then incorporating it into the nucleic acid with an enzyme such as terminal deoxynucleotidyl transferase, T4 RNA ligase, or DNA polymerase. In this case, the reactive group is contained in the nucleotide.

If the agent is RNA, the diol of ribose can be cleaved by periodate oxidation, and the reactive aldehyde group produced can be used to bind the chelating agent with the reactive group to the agent. In this case, the reactive group is a hydrazide group.

The chelating agent with a reactive group introduced can be prepared by methods known to those skilled in the art, such as synthesis by chemical synthesis.

In a preferred aspect, if the agent has an amino group, the chelating agent with a reactive group is DOTA with a reactive group, and the reactive group can be an ITC group. If the agent is a nucleic acid, it is preferable to bind the chelating agent having an amino group as the reactive group to the carboxyl group of the agent using a carbodiimide cross-linking agent.

In the present invention, "non-radioactive substance" refers to a substance having no radioactivity, that can be chelated by a chelating agent. The non-radioactive substance is typically a metal. Examples of the metal include lithium, beryllium, sodium, magnesium, aluminum, potassium, calcium, scandium, titanium, vanadium, chromium, manganese, iron, cobalt, nickel, copper, zinc, gallium, rubidium, strontium, yttrium, zirconium, niobium, molybdenum, ruthenium, rhodium, palladium, silver, cadmium, indium, tin, cesium, barium, lanthanum, cerium, praseodymium, neodymium, samarium, europium, gadolinium, terbium, dysprosium, holmium, erbium, thulium, ytterbium, lutetium, hafnium, tantalum, tungsten, rhenium, osmium, iridium, platinum, gold, mercury, thallium, lead, bismuth, and thorium. In a preferred aspect, the non-radioactive substance is indium or europium, and more preferably indium.

In step (i), a non-radioactive substance is chelated by a chelating agent having a reactive group. Usually, this reaction can be performed by bringing the non-radioactive substance into contact with the chelating agent in a suitable solvent. The conditions of the chelation reaction, such as the concentration of the non-radioactive substance and the chelating agent, the solvent used, pH, contact time, and temperature, can be appropriately set according to the type of non-radioactive substance and chelating agent.

In a preferred aspect, the chelating agent that chelated the non-radioactive substance can be purified after the chelation reaction. This allows to remove the non-radioactive substances that have not been chelated. For the purification, techniques generally known to those skilled in the art can be used, and examples thereof include purification by column chromatography, recrystallization, and the like.

In step (ii), the chelating agent that chelated the non-radioactive substance in step (i) is bound to the agent via the reactive group. The conditions for this binding reaction can be appropriately set according to the combination of the reacting group and the agent. The concentration of the agent is preferably higher than 0.01 mg/mL. For example, if the reactive group is an ITC group and the agent contains an amino group, the chelating agent with the reactive group and the agent can be mixed in a buffer such as phosphate buffered saline and left standing at room temperature to bind the chelating agent to the agent.

In a preferred aspect, the agent to which the chelating agent is bound (i.e., the agent labeled with a non-radioactive substance) can be purified after the binding reaction. This allows to remove the chelating agent that has not bound to the agent. For the purification, techniques generally known to those skilled in the art can be used, and examples thereof include purification by desalting columns, gel filtration chromatography, ultrafiltration, dialysis, and the like.

The composition containing the agent labeled with a non-radioactive substance by the Method I of the present invention is used in pharmacokinetic evaluation that do not use RI. Therefore, in another aspect, the present invention provides a method for producing a composition for pharmacokinetic evaluation comprising an agent labeled with a non-radioactive substance, the method comprising labeling the agent with a non-radioactive substance by the Method I of the present invention, and a composition for pharmacokinetic evaluation produced by the Method. Using the composition for pharmacokinetic evaluation, it is possible to accurately measure the agent concentration in a biological sample by spectrometry or mass spectrometry (such as ICP-MS), and to appropriately evaluate the pharmacokinetics.

The composition for pharmacokinetic evaluation can contain an appropriate solvent in addition to the agent labeled with a non-radioactive substance. Examples of such solvent include phosphate buffered saline; Tris buffered saline; 20 mM His-HCl, 150 mM NaCl, pH 6.0 buffer; 20 mM histidine-aspartate buffer containing 150 mM arginine-aspartate and 0.5 mg/mL kolliphor P188, pH 6.0; Hank's balanced salt solution (HBSS); and 10% DMSO/60% PEG300 in saline. If the agent is an antibody or a peptide compound, the solvent may contain 0.05% tween 20.

A pharmaceutically acceptable carrier may be added to the composition for pharmacokinetic evaluation. A pharmaceutically acceptable carrier means a component other than the agent, that is non-toxic to the subject of administration of the agent. Examples of pharmaceutically acceptable carriers include buffers, excipients, stabilizers, and preservatives.

### II. Method for Measuring Pharmacokinetics of Agent

The second aspect of the present invention relates to a method for measuring the pharmacokinetics of an agent (hereinafter also referred to as the Method II of the present invention), the method comprising:
(i) a step of labeling the agent with a non-radioactive substance by the Method I of the present invention,
(ii) a step of administering the agent labeled in step (i) to a non-human animal, and
(iii) a step of collecting a biological sample from the non-human animal after administration of the agent in step (ii) and measuring the content of the non-radioactive substance in the biological sample.

The step (i) is performed as described in I above.

For the administration to a non-human animal in step (ii), the agent labeled with a non-radioactive substance in step (i) is dissolved in an appropriate solvent. Examples of such solvent include phosphate buffered saline; Tris buffered saline; 20 mM His-HCl, 150 mM NaCl, pH 6.0 buffer; 20 mM histidine-aspartate buffer containing 150 mM arginine-aspartate and 0.5 mg/mL kolliphor P188, pH 6.0; Hank's balanced salt solution (HBSS); and 10% DMSO/60% PEG300 in saline. When administering an antibody or a peptide compound, the solvent may contain 0.05% tween 20. Pharmaceutically acceptable carriers such as buffers, excipients, stabilizers, and preservatives may be added to the solvent.

The non-human animal in step (ii) is not particularly limited as long as it is an animal other than a human and can be used for pharmacokinetic evaluation, and examples thereof include mammals except humans (monkeys, miniature pigs, rats, mice, rabbits, dogs, guinea pigs, etc.). In a preferred aspect, the non-human animal can be a mouse. The mouse may be, for example, a cancer-bearing mouse (such as a xenograft transplantation mouse where a human cancer cell line is transplanted into an immunocompromised mouse, and a syngeneic transplantation mouse where mouse cancer cells are transplanted into a fully immunocompetent syngeneic mouse), a pathological model mouse, or a transgenic mouse. Commercially available non-human animals may be used.

The "administration" in step (ii) can be appropriately selected according to the type of agent, and examples thereof include intravenous administration (i.v.), subcutaneous administration (s.c.), peroral administration (p.o.), intraperitoneal administration (i.p.), intramuscular administration (i.m.), intratumoral administration (i.t.), transpulmonary administration, and intranasal administration. In a preferred aspect, if the agent is an antibody, the administration of the agent can be intravenous administration. The administration of the agent may be a single administration, or repeated a plurality of times if necessary. The dosage and interval of administration can be appropriately adjusted according to the type of agent.

In step (iii), the biological sample is collected from a non-human animal after administration of the agent. The biological sample includes any tissues and organs, such as blood, plasma, serum, bile, urine, feces, brain, testis, ovary, lung, heart, stomach, ileum, large intestine, jejunum, kidney, liver, spleen, pancreas, muscle, and skin. The biological sample can be collected by a method usually used according to the type of sample. For example, blood can be collected from a non-human animal by venous blood collection. Serum can be prepared by collecting blood in a blood collection tube without anticoagulants, then allowing blood clots to agglutinate and removing the clots by centrifugation. Plasma can be prepared by collecting blood in a blood collection tube containing an anticoagulant such as heparin, then removing the blood cells by centrifugation. Solid tissues or solid organs can be collected by methods using a puncture needle, methods of obtaining by surgical incision, and the like.

The biological sample is collected after a given amount of time has elapsed after administering the agent. The time between the administration of the agent and collection can be appropriately set according to the type of agent, the pharmacokinetics to be evaluated, and the like, and can be set to, for example, several minutes to several days. For example, it can be set to 5 minutes to 28 days for antibodies, 2 minutes to 7 days for peptide compounds, 2 minutes to 14 days for nucleic acids, and 5 minutes to 14 days for cells. The number of times a biological sample is collected is appropriately set according to the pharmacokinetics to be evaluated, and may be once or a plurality of times. For example, if measuring the changes in the amount of agent present in a particular tissue or organ (e.g., changes in blood concentration), the biological sample is collected a plurality of times over time. If measuring the distribution of the agent in a tissue or organ, the biological sample is usually collected once at a particular time point after administration of the agent.

The content of the non-radioactive substance in the biological sample can be measured by methods generally used in the field of inorganic analysis. From the viewpoint of measurement efficiency and the like, it is preferable to measure by spectrometry or mass spectrometry.

Examples of spectrometry include atomic absorption spectrometry, ICP emission spectrometry, and X-ray fluorescence analysis.

Ionization methods in mass spectrometry can be appropriately selected according to the type of non-radioactive substance, sample morphology, and the like, and examples thereof include plasma ionization, electron ionization (EI), chemical ionization (CI), atmospheric pressure chemical ionization (APCI), electrospray ionization (ESI), and matrix-assisted laser desorption/ionization (MALDI). Examples of plasma ionization include ionization by inductively coupled plasma (ICP), ionization by microwave-induced plasma (MIP), and ionization by glow discharge (GD).

Examples of the mass spectrometry include plasma ionization mass spectrometry, electron ionization (EI) mass spectrometry, chemical ionization (CI) mass spectrometry, atmospheric pressure chemical ionization (APCI) mass spectrometry, electrospray ionization (ESI) mass spectrometry, matrix-assisted laser desorption/ionization (MALDI) mass spectrometry, laser desorption mass spectrometry (LDMS), secondary ion mass spectrometry (SIMS), spark source mass spectrometry (SSMS), and thermal ionization mass spectrometry (TIMS). In a preferred aspect, the mass spectrometry is plasma ionization mass spectrometry, more preferably inductively coupled plasma (ICP) mass spectrometry, microwave-induced plasma (MIP) mass spectrometry, or glow discharge (GD) mass spectrometry, and most preferably ICP mass spectrometry.

The biological sample may be pretreated depending on the method and equipment used to measure the content. As a pretreatment, treatments generally used in inorganic analysis can be used, and examples thereof include chemical treatments such as acidolysis, microwave treatment, combustion, pressurized acidolysis, alkali fusion, dry ashing, extraction, elution, and co-precipitation. For example, if the content is measured by ICP mass spectrometry, the pretreatment can be performed by adding nitric acid to the biological sample and doing a microwave treatment.

From the content of the non-radioactive substance in the biological sample, the relative content of the agent, such as the distribution of the agent in different organs can be known, and thereby the pharmacokinetics of the agent can be measured and evaluated.

Alternatively, the absolute content of the agent can be known by calculating the labeling efficiency of the non-radioactive substance to the agent and thereby converting the content of the non-radioactive substance to the content of the agent. The pharmacokinetics can thereby be measured and evaluated. Therefore, in a preferred aspect, for an agent labeled with a non-radioactive substance in step (i), the labeling efficiency of the non-radioactive substance to the agent can be measured. The labeling efficiency is the percentage of the number of molecules of non-radioactive substance bound per molecule of the agent if the agent is a molecule, or the mole number (mol) of non-radioactive substance bound per agent if the agent is a cell. For a solution containing an agent labeled with a non-radioactive substance, the concentration of the non-radioactive substance and the concentration of the agent are each measured to calculate the labeling efficiency. If the agent is a molecule, the labeling efficiency is determined by calculating the value (%) of the concentration of non-radioactive labeling (mol/L) / agent concentration (mol/L) x 100 in the solution. If the agent is a cell, the labeling efficiency is determined by calculating the value (mol/cell) of the concentration of non-radioactive labeling (mol/L) / agent concentration (cell/L) in the solution. The concentration of the non-radioactive substance can be measured by the same method as in step (iii). The concentration of the agent can be measured using an appropriate method according to the type of agent. For example, if the agent is a protein such as an antibody, methods such as the BCA method, Bradford method, Lowry method, and ultraviolet spectrophotometric method can be used. If the agent is a peptide compound, methods such as amino acid analysis and ultraviolet spectrophotometric method can be used. If the agent is a nucleic acid, methods such as absorption spectrometry and fluorometric analysis can be used. If the agent is a cell, methods such as FACS and cell counting can be used.

In the Method II of the present invention, a process of quantifying the pharmacokinetics can further be included to evaluate the pharmacokinetics. To "quantify the pharmacokinetics" includes calculating any of the PK parameters described in I above, and the methods for calculating these are well known to those skilled in the art.

The administration in step (ii), the collection of the biological sample in step (iii), and the measurement and evaluation of the pharmacokinetics can be performed according to known guidelines such as the "Guidelines for Nonclinical Pharmacokinetic Studies" (PMSB/ELD Notification No. 496, June 26, 1998).

In one embodiment, in the Method II of the present invention, the two or more agents can be labeled with different non-radioactive substances and administered to a single non-human animal. This allows to reduce the number of laboratory animals used when measuring the pharmacokinetics of two or more agents. The spectrometry or mass spectrometry described above is a method that can simultaneously measure different non-radioactive substances and can measure the content of different non-radioactive substances in a biological sample at once. The agents can be administered simultaneously to a non-human animal, where "administered simultaneously" includes administering separately within a time period sufficiently short relative to the time between administration of the agent and collection of the biological sample, for example, within 1 minute to several hours (such as 5 minutes, 10 minutes, 30 minutes, or 1 hour).

In a preferred aspect of the Method II of the present invention, the pharmacokinetics can be organ distribution. In this case, the biological sample is an organ, and the method further comprises (iv) a step of determining the distribution of the agent in the organ based on the content of the non-radioactive substance measured in step (iii) (hereinafter also referred to as the Method II-1 of the present invention).

In the Method II-1 of the present invention, the organ is as described for the Method II of the present invention, and examples thereof include at least one (e.g., two or more) selected from the group consisting of blood, plasma, red blood cells, salivary glands, thyroid gland, esophagus, ileum, large intestine, jejunum, stomach, axillary lymph nodes, thymus gland, lungs, heart, aorta, liver, pancreas, spleen, adrenal gland, white fat, mesenteric lymph nodes, testes, prostate, bladder, kidneys, muscles, sciatic nerve, bone marrow, skin, brown fat, harderian gland, eyes, cerebellum, cerebrum, medulla oblongata, pituitary gland, spinal cord, nonglandular stomach, glandular stomach, small intestine, mammary glands, ovaries, uterus, placenta, amniotic fluid, fetus, and cancers (in subcutaneously implanted cancer-bearing mice), preferably at least one (e.g., two or more) selected from the group consisting of blood, plasma, testes, ileum, large intestine, jejunum, stomach, liver, lungs, kidneys, spleen, heart, thigh muscles, and skin.

In step (iv), the distribution of the agent in the organ is determined from the content of the agent estimated from the content of the non-radioactive substance, or the content of the agent converted using the labeling efficiency. This can provide an index for selecting agents with the desired organ distribution.

The Method II-1 of the present invention may further comprise (v) a step of quantifying the pharmacokinetics based on the distribution determined in step (iv). Here, quantification means to calculate the numerical value relating to the pharmacokinetic parameters mentioned above, and examples thereof includes calculating the value expressed as a percentage (expressed as "% of Dose") of the amount distributed in each organ or tissue divided by the dose to express the distribution pattern of the agent throughout the body, calculating the concentration (expressed, for example, as "µg/g tissue") obtained by dividing the amount distributed in each organ or tissue by the weight of that organ or tissue to express the amount of distribution per unit weight of each organ or tissue, calculating the "Tissue/Plasma ratio", a value obtained by dividing the concentration in an organ or tissue by the blood concentration to evaluate the degree of concentration in the organ or tissue relative to the blood concentration, calculating uptake clearance by integration plots to evaluate shortterm uptake over time, and calculating the tissue-plasma partition coefficient (Kp) which expresses the transfer to an organ or tissue from the concentration of the administered product in the organ or tissue by applying a physiologically-based pharmacokinetic (PBPK) model. These parameters are calculated according to methods known in the art.

In another preferred aspect of the Method II of the present invention, the pharmacokinetics can be the changes in blood concentration. In this case, the biological sample is blood, plasma, or serum, step (iii) is a step of collecting the biological sample from the non-human animal twice or more over time after administration of the agent in step (ii) and measuring the content of the non-radioactive substance in the biological sample, and the method further comprises (iv) a step of determining the changes in blood concentration of the agent based on the content of the non-radioactive substance measured in step (iii) (hereinafter also referred to as the Method II-2 of the present invention).

In the Method II-2 of the present invention, the biological sample is preferably plasma.

The administration of the agent in step (ii) is usually performed only once.

In step (iii), the number of times the biological sample is collected can be appropriately adjusted according to the type of agent and the like, and can be, for example, three times or more, four times or more, five times or more, or six times or more. The interval between each collection can be appropriately adjusted according to the type of agent and the like, and for example, can be set to several minutes to several days and need not be constant.

In step (iv), the changes in blood concentration of the agent are determined from the content of the agent estimated from the content of the non-radioactive substance, or the content of the agent converted using the labeling efficiency. This can provide an index for selecting agents with the desired changes in blood concentration.

The Method II-2 of the present invention may further comprise (v) a step of quantifying the pharmacokinetics based on the changes in blood concentration determined in step (iv). Here, quantification includes calculating PK parameters based on the changes in blood concentration, such as clearance (CL), volume of distribution (Vd or V), area under the blood concentration-time curve (AUC), maximum blood concentration (Cmax), time to reach maximum blood concentration (Tmax), and blood concentration half-life (t_{1/2}). These parameters are calculated according to methods known in the art.

According to the Method II of the present invention, it is possible to evaluate the pharmacokinetics of an agent as accurately as if RI were used, without using RI.

### III. Method for Screening Agents Having Desired Pharmacokinetics

The third aspect of the present invention relates to a method for screening agents having a desired pharmacokinetics (hereinafter also referred to as the Method III of the present invention), the method comprising:
(i) a step of measuring the pharmacokinetics of two or more candidate agents by the Method II of the present invention, and
(ii) a step of comparing the pharmacokinetics of the candidate agents measured in step (i) and selecting an agent exhibiting the desired pharmacokinetics.

Step (i) can be performed according to I and II above. In step (i), the two or more candidate agents are each selected from the agents listed in I above.

The selection of an agent exhibiting the desired pharmacokinetics in step (ii) can be done by quantifying the pharmacokinetics and then comparing the numerical values for each agent. If the pharmacokinetics is organ distribution, the agent exhibiting the organ distribution expected to exert the most pharmacological effect can be selected.

In one embodiment, in the Method III of the present invention, the two or more candidate agents can be labeled with different non-radioactive substances and administered to a single non-human animal. This allows to reduce the number of laboratory animals used for screening. The spectrometry or mass spectrometry described in II above is a method that can simultaneously measure different non-radioactive substances and can measure the content of different non-radioactive substances in a biological sample at once. The agents can be administered simultaneously, where "administered simultaneously" includes administering separately within a time period sufficiently short relative to the time between administration of the agent and collection of the biological sample, for example, within 1 minute to several hours (e.g., 5 minutes, 10 minutes, 30 minutes, or 1 hour).

According to the Method III of the present invention, it is possible to efficiently select an agent having the desired pharmacokinetics from a large number of candidate agents, without using RI.

### IV. Composition for Pharmacokinetic Evaluation

The fourth aspect of the present invention relates to a composition for pharmacokinetic evaluation (hereinafter also referred to as Composition IV of the present invention),
the composition comprising an agent to which a chelating agent is bound,
wherein a non-radioactive substance is chelated by the chelating agent.

There is no known method for labeling an agent with a non-radioactive substance in a way suitable for pharmacokinetic evaluation, and there are no known examples of compositions containing an agent labeled with a non-radioactive substance, such as the composition IV of the present invention, being used for pharmacokinetic evaluation. The present invention makes it possible for the first time to provide a composition for pharmacokinetic evaluation comprising an agent labeled with a non-radioactive substance.

For the "pharmacokinetics," "chelating agent," "agent," and "non-radioactive substance" in the Composition IV of the present invention, the above description relating to the Method I of the present invention is used.

The bond between the chelating agent and the agent is a covalent bond, and is appropriately selected according to the type of agent and the type of reaction used to bind the agent to the chelating agent. The bond is preferably not easily cleaved in the living body. Examples of such bonds include those described with respect to the Method I of the present invention. In a preferred aspect, the chelating agent may be bound to the agent by a chemical bond with an amino group or a thiol group of the agent. Examples of such bonds include the bond between an amino group in the agent and an NHS ester or ITC group, more preferably an ITC group, introduced into the chelating agent, and the bond between a thiol group in the agent and a maleimide group introduced into the chelating agent. If the agent is a nucleic acid, it is preferable to bind the chelating agent with an amino group introduced to the carboxyl group of the nucleic acid using a carbodiimide cross-linking agent. In this case, the chelating agent and the nucleic acid are bound by an amide bond.

The agent bound to the chelating agent chelating the non-radioactive substance, which is contained in the Composition IV of the present invention, is not particularly limited, and can be prepared according to, for example, the Method I of the present invention.

The composition for pharmacokinetic evaluation can contain an agent labeled with a non-radioactive substance, as well as an appropriate solvent. Examples of such solvent include phosphate buffered saline; Tris buffered saline; 20 mM His-HCl, 150 mM NaCl, pH 6.0 buffer; 20 mM histidine-aspartate buffer containing 150 mM arginine-aspartate and 0.5 mg/mL kolliphor P188, pH 6.0; Hank's balanced salt solution (HBSS); and 10% DMSO/60% PEG300 in saline. If the agent is an antibody or peptide compound, the solvent may contain 0.05% tween 20.

A pharmaceutically acceptable carrier may be added to the composition for pharmacokinetic evaluation. A pharmaceutically acceptable carrier means a component other than the agent, that is non-toxic to the subject of administration of the agent. Examples of pharmaceutically acceptable carriers include buffers, excipients, stabilizers, and preservatives.

The Composition IV of the present invention can be used to measure pharmacokinetics as described with respect to the Method II of the present invention, to screen agents with the desired pharmacokinetics as described with respect to the Method III of the present invention, and the like.

### V. Method for Measuring Pharmacokinetics of Agent

The fifth aspect of the present invention relates to a method for measuring the pharmacokinetics of an agent (hereinafter also referred to as the Method V of the present invention), the method comprising:
(i) a step of administering an agent labeled with a non-radioactive substance to a non-human animal, and
(ii) a step of collecting a biological sample from the non-human animal after administration of the agent in step (i) and measuring the content of the non-radioactive substance in the biological sample.

For the "pharmacokinetics" and "agent" in the Method V of the present invention, the above description relating to the Method I of the present invention is used.

"Non-radioactive substance" in the Method V of the present invention refers to a substance having no radioactivity, the content of which can be measured by spectrometry or mass spectrometry, and includes metals, non-metals, and semi-metals. Examples of the metal include lithium, beryllium, sodium, magnesium, aluminum, potassium, calcium, scandium, titanium, vanadium, chromium, manganese, iron, cobalt, nickel, copper, zinc, gallium, rubidium, strontium, yttrium, zirconium, niobium, molybdenum, ruthenium, rhodium, palladium, silver, cadmium, indium, tin, cesium, barium, lanthanum, cerium, praseodymium, neodymium, samarium, europium, gadolinium, terbium, dysprosium, holmium, erbium, thulium, ytterbium, lutetium, hafnium, tantalum, tungsten, rhenium, osmium, iridium, platinum, gold, mercury, thallium, lead, bismuth, and thorium. Examples of non-metals include phosphorus, sulfur, selenium, and iodine. Examples of semi-metals include boron, silicon, germanium, arsenic, antimony, and tellurium. In a preferred aspect, the non-radioactive substance is a metal, more preferably indium or europium, and most preferably indium.

For labeling an agent with a non-radioactive substance, methods known in the art can be appropriately used, according to the type of non-radioactive substance and agent. As such methods, methods known in the art, such as those used for labeling with radioisotopes, can be used in addition to methods using a chelating agent such as the Method I of the present invention. For example, when labeling an agent with iodine, the chloramine-T method, IODO-GEN method, Bolton-Hunter method, and lactoperoxidase method used for labeling with radioiodine (see, for example, Clinical Chemistry, Vol. 2, No. 4 (1974) 379-385) can be used. If the agent is a protein, the agent can be labeled with selenium by preparing a protein in which cysteine is replaced with selenocysteine (see, for example, WO2003/029469).

For the description of the administration to a non-human animal of step (i) in the Method V of the present invention, the above description relating to the step (ii) in the Method II of the present invention is used. The step (ii) in the Method V of the present invention corresponds to the step (iii) in the Method II of the present invention, and the above description relating to the Method II of the present invention is used.

In a preferred aspect of the Method V of the present invention, the pharmacokinetics can be organ distribution. In this case, the biological sample is an organ, and the method further comprises (iii) a step of determining the distribution of the agent in the organ based on the content of the non-radioactive substance measured in step (ii) (hereinafter also referred to as the Method V-1 of the present invention). The Method V-1 of the present invention corresponds to the Method II-1 of the present invention, and the description thereof is used.

In another preferred aspect of the Method V of the present invention, the pharmacokinetics can be the changes in blood concentration. In this case, the biological sample is blood, plasma, or serum, step (ii) is a step of collecting the biological sample from the non-human animal twice or more over time after administration of the agent in step (i) and measuring the content of the non-radioactive substance in the biological sample, and the method further comprises (iii) a step of determining the changes in blood concentration of the agent based on the content of the non-radioactive substance measured in step (ii) (hereinafter also referred to as the Method V-2 of the present invention). The Method V-2 of the present invention corresponds to the Method II-2 of the present invention, and the description thereof is used.

### VI. Method for Screening Agents Having Desired Pharmacokinetics

The sixth aspect of the present invention relates to a method for screening agents having a desired pharmacokinetics (hereinafter also referred to as the Method VI of the present invention), the method comprising:
(i) a step of measuring the pharmacokinetics of two or more candidate agents by the Method V of the present invention, and
(ii) a step of comparing the pharmacokinetics of the candidate agents measured in step (i) and selecting an agent exhibiting the desired pharmacokinetics.

The Method VI of the present invention corresponds to the Method III of the present invention, except that the Method V of the present invention is used in step (i) instead of the Method II of the present invention, and the description thereof is used.

In the Methods II, III, V, and VI of the present invention, internal standards can also be used to quantify the content of the non-radioactive substance in the sample.

The method of internal standards is a quantitative method in which a given amount of an internal standard substance is added to each of a standard sample and an unknown sample, and the concentration of the target component is determined based on the relationship with the concentration ratio of the target component and the internal standard substance. Using this method allows to correct for errors in the volume injected into the analytical instrument, errors occurring in the preparation of plasma or organs as samples for measurement, and the like.

The internal standard can be appropriately selected from substances differing from the non-radioactive substance, according to the method of analysis used to measure the content of the non-radioactive substance. As an internal standard, a substance that is not contained in the atmosphere or, if contained in the atmosphere, its effect is negligible considering the concentration added to the measurement sample, can be used. Examples of elements used as internal standards include beryllium, lithium, cobalt, bismuth, thallium, holmium, indium, rhodium, scandium, terbium, and yttrium.

Measurement by the method of internal standards can be performed, for example, as follows. A sample containing an agent labeled with a non-radioactive substance (such as the antibody denosumab labeled with indium), and for which the concentration of the non-radioactive substance has been previously quantified is used as the standard sample, and a sample containing an agent labeled with a non-radioactive substance, but for which the concentration of the non-radioactive substance has not been measured is used as the unknown sample. To each of these, a given amount of internal standard substance is added. To create a calibration curve, a plurality of standard samples of different concentrations are prepared and analyzed to obtain a calibration curve for the concentration ratios and peak area ratios of the non-radioactive substance to the internal standard substance. The peak area ratio for the unknown sample, to which the same amount of internal standard substance as the standard sample is added, is then measured and applied to the above calibration curve to obtain the concentration of the non-radioactive substance in the unknown sample.

The present invention will be described in more detail with reference to Examples.

### EXAMPLES

### [Example 1] Measurement of changes in blood concentration

### 1. Synthesis of labeling reagent

In-p-SCN-Bn-DOTA was obtained by contract synthesis from Macrocyclics, Inc.

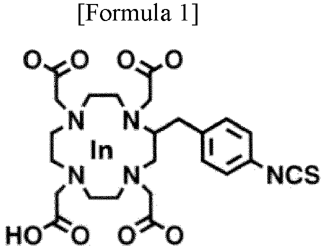

### 2. Labeling

A Zeba spin desalting column (87768, Thermo Fisher Scientific) was used to replace the solvent of cetuximab (Erbitux injection, Merck Biopharma) with PBS (phosphate buffered saline). To a solution containing 0.01 µmol of cetuximab, 0.53 µmol of In-p-SCN-Bn-DOTA dissolved in DMSO was added, and the mixture was stirred then left standing at room temperature for 4 hours. From the obtained reaction solution, labeled cetuximab (In-DOTA-labeled cetuximab) was purified by a gel filtration column. PBS was used as the eluent.

### 3. Calculation of labeling efficiency using ICP-MS

Nitric acid was added to the In-DOTA-labeled cetuximab prepared in 2 above, which was then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The measured value of the sample solution was compared with the calibration curve to calculate the concentration of indium in the solution. The antibody concentration in the sample solution was also measured using a spectrophotometer. The value (%) calculated from indium concentration (mol/L) / antibody concentration (mol/L) x 100 was used as the labeling efficiency. The labeling efficiency was 45%.

### 4. Animal administration and blood collection

The In-DOTA-labeled cetuximab prepared in 2 above was administered intravenously to C57BL6J mice as a single dose of 1 mg/kg through the tail vein. Blood samples were collected a plurality of times over time, from 5 minutes to 7 days after administration. The obtained blood was centrifuged to separate the plasma. The plasma was stored in a freezer set to -20°C or less until measurement.

### 5. Measurement of plasma concentration in mice using ICP-MS

Nitric acid was added to the mouse plasma samples, which were then decomposed in a microwave sample pretreatment device (ETHOS 1, Milestone). A solution made to constant volume with ultrapure water was used as the sample solution and measured with an ICP mass spectrometer. An indium standard solution was also prepared and subjected to ICP-MS analysis to create a calibration curve. The measured value of the sample solution was compared with the calibration curve to calculate the concentration of indium in the solution. From the labeling efficiency and indium concentration, the antibody concentration in the mouse plasma samples was calculated.

### 6. Measurement of plasma concentration in mice using LBA

2 µL of mouse plasma sample was diluted 100-fold with TBS-T and used as the plasma sample for measurement. To create the calibration curve, In-DOTA-labeled cetuximab was diluted with 1% (v/v) plasma/TBS-T (plasma from mice that were not given indium-DOTA-labeled cetuximab, diluted with TBS-T to 1% (by volume)), and standard solutions with concentrations of In-DOTA-labeled cetuximab of 2500, 833.3, 277.8, 92.6, 30.9, 10.3, and 3.4 ng/mL were prepared. Biotin-labeled anti-human κ antibody (AS76-B, Antibody Solutions) was used as the solid phase antibody. Mouse anti-human Fc antibody (9040-01, SouthernBiotech) labeled with Alexa647 was used as the detection antibody. Each solution was set in an automated ELISA system Gyrolab xP workstation and injected into a Bioaffy 200 CD (Gyros AB.) to measure the content of In-DOTA labeled cetuximab in the plasma sample for measurement and to calculate the plasma concentration.

### 7. Results

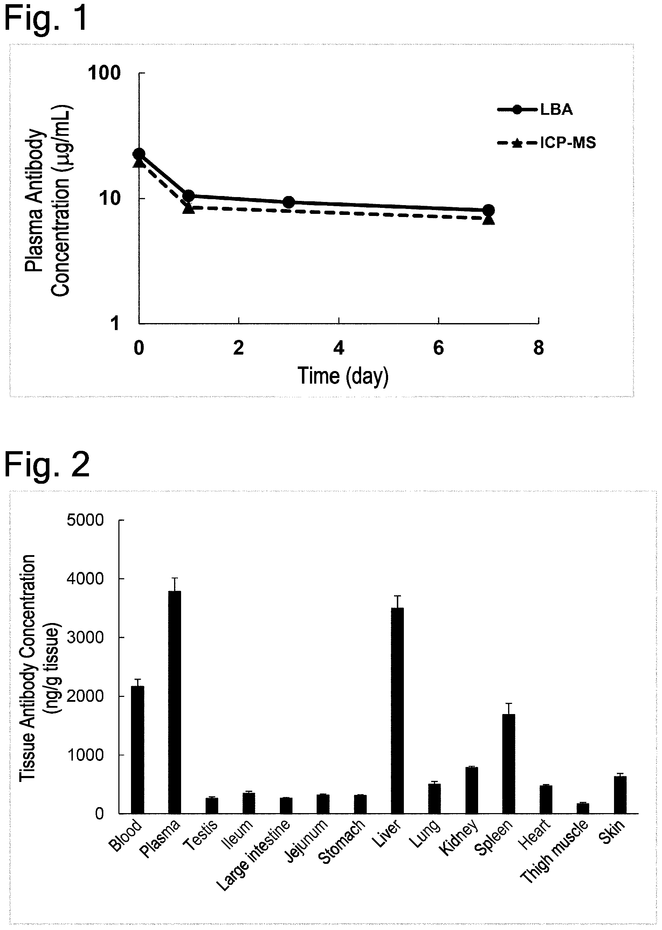

The results are shown in Figure 1. The changes in plasma antibody concentration of In-DOTA-labeled cetuximab measured by ICP-MS and LBA were comparable. The results of a non-compartmental analysis of the changes in plasma antibody concentration are shown in Table 1. The clearance ("Clearance" in Table 1) was 14.3 mL/day/kg with LBA and 16.9 mL/d/kg with ICP-MS. This suggests that the changes in plasma concentration of cetuximab can be evaluated by measuring the plasma concentration of indium in mice given In-DOTA-labeled cetuximab by ICP-MS.

**[Table 1]**

| NCA Parameters for Changes in Plasma Antibody Concentration | | | | | |
|---|---|---|---|---|---|
| | | LBA | | ICP-MS | |
| | | Mean | S.D. | Mean | S.D. |
| Vss | mL/kg | 88.8 | 1.7 | 111 | 15.6 |
| Clearance | mL/day/kg | 14.3 | 0.5 | 16.9 | 0.2 |

### [Example 2] Measurement of organ distribution

### 1. Labeling

A Zeba spin desalting column was used to replace the solvent of cetuximab (Erbitux injection, Merck Biopharma) with PBS. 0.81 µmol of In-p-SCN-Bn-DOTA dissolved in DMSO was added to a solution containing 0.016 µmol of cetuximab, and the mixture was stirred then left standing at room temperature for 4 hours. From the obtained reaction solution, labeled cetuximab (In-DOTA-labeled cetuximab) was purified using a gel filtration column. PBS-T was used as the eluent.

### 2. Calculation of labeling efficiency using ICP-MS

The labeling efficiency was calculated for the In-DOTA-labeled cetuximab prepared in 1 above as in Example 1. The labeling efficiency was 108%.

### 3. Animal administration

The In-DOTA-labeled cetuximab prepared in 1 above was administered intravenously to C57BL6J mice as a single dose of 1 mg/kg through the tail vein. Whole blood was collected from the abdominal vena cava under isoflurane anesthesia at 24 hours after administration, followed by harvest of tissues (liver, lung, kidney, heart, spleen, thigh muscle, skin, stomach, jejunum, ileum, large intestine, and testis). The obtained blood was centrifuged to separate the plasma. All organs, blood and plasma were stored in a freezer set to -20°C or less until measurement.

### 4. Measurement of blood, plasma and tissue concentration in mice using ICP-MS

Nitric acid was added to the mouse blood, plasma, and tissue (liver, lung, kidney, heart, spleen, thigh muscle, skin, stomach, jejunum, ileum, large intestine, and testis) samples, which were then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The measured value of the sample solution was compared with the calibration curve to calculate the concentration of indium in the solution. From the labeling efficiency and indium concentration, the antibody concentration in the mouse blood and plasma samples was calculated. From the labeling efficiency, indium concentration, and tissue weight, the antibody concentration in each mouse organ sample was calculated.

### 5. Results

The results are shown in Figure 2. Indium was detected not only in blood and plasma, but also in each tissue (liver, lung, kidney, heart, spleen, thigh muscle, skin, stomach, jejunum, ileum, large intestine, and testis), and the tissue antibody concentration could be calculated. This suggests that the antibody concentration in the organs of mice given In-DOTA-labeled cetuximab can be evaluated by ICP-MS.

### [Comparative Example 1] Measurement of changes in blood concentration of antibody labeled with indium by conventional method

### 1. Labeling

To a solution containing 0.03 µmol of cetuximab, 0.3 µmol of DTPA anhydride dissolved in DMSO was added, and the mixture was stirred then left standing at room temperature for 30 minutes. From the obtained reaction solution, DTPA-bound cetuximab (DTPA-cetuximab) was purified by a gel filtration column. 0.1 M acetate buffer + 0.05% tween20 (pH 6.0) was used as the eluent. To 0.028 µmol of DTPA-cetuximab, 0.084 µmol of indium chloride dissolved in 1M acetate buffer and saline was added. The mixture was stirred then left standing at room temperature for 30 minutes. To the reaction solution, 0.084 µmol of EDTA (ethylenediaminetetraacetic acid) dissolved in 0.1 M acetate buffer was added. The mixture was stirred then left standing at room temperature for 30 minutes. From the obtained reaction solution, DTPA-cetuximab labeled with indium (In-labeled DTPA-cetuximab) was purified by a gel filtration column. PBS-T was used as the eluent.

### 2. Calculation of labeling efficiency using ICP-MS

The labeling efficiency was calculated for the In-labeled DTPA-cetuximab prepared in 1 above as in Example 1. The labeling efficiency was 182%.

### 3. Animal administration and blood collection

The In-labeled DTPA-cetuximab prepared in 1 above was administered intravenously to C57BL6J mice as a single dose of 10 mg/kg through the tail vein. Blood samples were collected a plurality of times over time, from 5 minutes to 7 days after administration. The obtained blood was centrifuged to separate the plasma. The plasma was stored in a freezer set to -20°C or less until measurement.

### 4. Measurement of plasma concentration in mice using ICP-MS

Mouse plasma samples were decomposed by treatment with nitric acid, followed by treatment with hydrogen peroxide, and evaporated to dryness. The dried solid dissolved in nitric acid by heating was used as a sample solution, and the indium in the solution was measured by ICP mass spectrometry (method of internal standards). A plurality of standard solutions with different indium concentrations were also prepared and subjected to ICP-MS analysis, and a calibration curve was created based on the data obtained. The measured value of the sample solution was compared with the calibration curve to calculate the concentration of indium in the solution. From the labeling efficiency and indium concentration, the antibody concentration in the mouse plasma samples was calculated.

### 5. Measurement of plasma concentration in mice using LBA

2 µL of mouse plasma sample was diluted 25-fold with TBS-T, then further diluted 16-fold with 4% plasma/TBS-T (final: 400-fold dilution), and used as the plasma sample for measurement. To create the calibration curve, In-labeled DTPA-cetuximab was diluted with 4% (v/v) plasma/TBS-T, and standard solutions with concentrations of In-labeled DTPA-cetuximab of 2500, 833.3, 277.8, 92.6, 30.9, 10.3, and 3.4 ng/mL were prepared. Biotin-labeled anti-human κ antibody (AS76-B, Antibody Solutions) was used as the solid phase antibody. Mouse anti-human Fc antibody (9040-01, SouthernBiotech) labeled with Alexa647 was used as the detection antibody. Each solution was set in an automated ELISA system Gyrolab xP workstation and injected into a Bioaffy 200 CD (Gyros AB.) to measure the content of In-labeled DTPA-cetuximab in the plasma sample for measurement and to calculate the plasma concentration.

### 6. Results

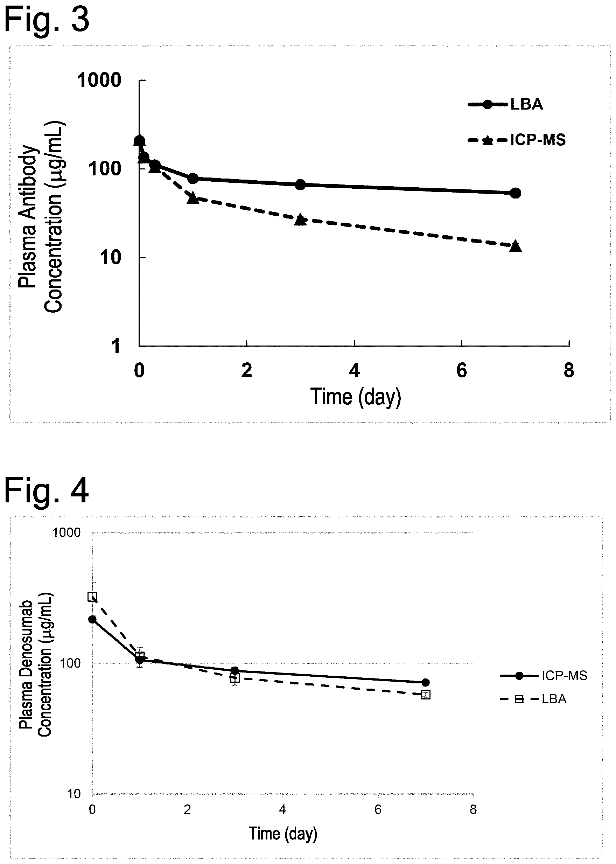

The results are shown in Figure 3. The plasma concentrations of In-labeled DTPA-cetuximab measured by ICP-MS showed faster disappearance than plasma concentrations of In-labeled DTPA-cetuximab measured by LBA. The results of a non-compartmental analysis of the changes in plasma antibody concentration are shown in Table 2. The clearance was 20.5 mL/d/kg with LBA and 41.4 mL/d/kg with ICP-MS, which is a large deviation.

**[Table 2]**

| NCA Parameters for Changes in Plasma Antibody Concentration | | | | | |
|---|---|---|---|---|---|
| | | LBA | | ICP-MS | |
| | | Mean | S.D. | Mean | S.D. |
| Vss | mL/kg | 118 | 10.1 | 142 | 12.0 |
| Clearance | mL/day/kg | 20.5 | 0.95 | 41.4 | 2.77 |

### [Example 3] PK of iodine-labeled denosumab in wild-type mice

### 1. Examination of labeling conditions

### (1) Labeling

A Zeba spin desalting column (40 K, 2 mL, Thermo Fisher Scientific) was used to replace the solvent of denosumab (Ranmark subcutaneous injection, Daiichi Sankyo) with PBS. The Iodogen method and Bolton-Hunter method were examined as methods of iodine labeling.

A. Iodogen: To an iodination tube (manufactured by Pierce), 28 nmol of sodium iodide dissolved in 1 mM sodium hydroxide solution and Tris buffer were added, then the mixture was stirred and allowed to react at room temperature for 6 minutes. The obtained reaction solution was added to a solution containing 1.33 nmol of denosumab, and the mixture was stirred and allowed to react at room temperature for 6 minutes. The obtained reaction solution was purified using PD-10 (manufactured by Cytiva). PBS was used as the eluent.

B. Bolton-Hunter: To 27 nmol of Sulfo-SHPP dissolved in DMSO, 267 nmol of sodium iodide dissolved in 1 mM sodium hydroxide solution and Chloramin-T dissolved in PBS were added, and the mixture was stirred. To the obtained mixed solution, a solution containing 1.33 nmol of denosumab was added, and the mixture was stirred and left standing on ice for 30 minutes. The obtained reaction solution was purified using PD-10 (manufactured by Cytiva). PBS + 0.2 M arginine was used as the eluent.

### (2) Calculation of labeling efficiency using ICP-MS

After adding sodium hypochlorite to the labeled antibody sample, iodine in the solution was measured by ICP mass spectrometry. An iodine standard solution was also prepared and subjected to ICP-MS analysis, and a calibration curve was created based on the data obtained. The obtained measured value was compared with the calibration curve to calculate the concentration of iodine in the solution. The iodine concentration in the labeled antibody solution of known concentration was measured, and the labeling efficiency was calculated from the result. The value (%) calculated from iodine concentration (mol/L) / antibody concentration (mol/L) x 100 was used as the labeling efficiency. The labeling efficiencies under each condition are shown in Table 3.

**[Table 3]**

| Labeling Efficiencies by Two Iodine Labeling Methods | | | |
|---|---|---|---|
| | Labeling Method | Equivalent Amount of Labeling Reagent (Equivalent) | Labeling Efficiency |
| A | lodogen | 21 | 2072% |
| B | Bolton-Hunter | 20 | 5568% |

### 2. Evaluation of PK of iodine-labeled denosumab by Iodogen method

### (1) Labeling

A Zeba spin desalting column (40 K, 2 mL, Thermo Fisher Scientific) was used to replace the solvent of denosumab (Ranmark subcutaneous injection, Daiichi Sankyo) with PBS. To an iodination tube (manufactured by Pierce), 2.8 µmol of sodium iodide dissolved in 1 mM NaOH solution and Tris buffer were added, then the mixture was stirred and allowed to react at room temperature for 6 minutes. The obtained reaction solution was added to a solution containing 13.3 nmol of denosumab, and the mixture was stirred and allowed to react at room temperature for 6 minutes. The obtained reaction solution was purified using PD-10 (manufactured by Cytiva). PBS + 0.2 M arginine was used as the eluent.

### (2) Calculation of labeling efficiency using ICP-MS

After adding sodium hypochlorite to the labeled antibody sample, iodine in the solution was measured by ICP mass spectrometry. An iodine standard solution was also prepared and subjected to ICP-MS analysis, and a calibration curve was created based on the data obtained. The obtained measured value was compared with the calibration curve to calculate the concentration of iodine in the solution. The iodine concentration in the labeled antibody solution of known concentration was measured, and the labeling efficiency was calculated from the result. The labeling efficiency was 4161%.

### (3) Animal administration and blood collection

The antibody labeled in (1) above was administered intravenously to C57BL6J mice as a single dose of 10 mg/kg through the tail vein. Blood samples were collected a plurality of times over time, from 5 minutes to 7 days after administration. Plasma was separated from the obtained blood by centrifugation. The plasma was stored in a freezer set to -20°C or less until measurement.

### (4) Measurement of iodine concentration in mouse plasma and calculation of antibody concentration using ICP-MS

After adding sodium hypochlorite to the mouse plasma sample, and making it to constant volume with ultrapure water, iodine in the solution was measured by ICP mass spectrometry. An iodine standard solution was also prepared and subjected to ICP-MS analysis, and a calibration curve was created based on the data obtained. The obtained measured value was compared with the calibration curve to calculate the concentration of iodine in the solution. From the labeling efficiency and iodine concentration in the mouse plasma samples, the antibody concentration in the samples was calculated.

### (5) Measurement of antibody concentration in mouse plasma using LBA

The mouse plasma samples were diluted 100-fold with 1% BSA in PBS-T, then further diluted 10-fold with 1% plasma/1% BSA in PBS-T (final: 1000-fold dilution), and used as the plasma sample for measurement. To create the calibration curve, iodine-labeled denosumab was diluted with 1% plasma/1% BSA in PBS-T, and diluted solutions of 1024, 512, 256, 128, 64, 32, and 16 ng/mL were prepared. Anti-human IgG antibodies (Bethyl) were added to sector plates (Meso scale discovery) as the solid phase antibody. Biotin-labeled anti-human antibodies (SouthernBiotech) were added as the detection antibody, and after incubation, Sulfo-tag-labeled streptavidin (Meso scale discovery) was added. After incubation, Read buffer (Meso scale discovery) was added and the content of the test substance was measured to calculate the concentration in plasma.

### (6) Results

The results of measuring the changes in average plasma concentration of iodine-labeled denosumab in C57BL6J mice by ICP-MS and LBA are shown in Figure 4. The changes in plasma concentration of iodine-labeled denosumab measured by ICP-MS were comparable to the changes in average plasma concentration of iodine-labeled denosumab measured by LBA.

Furthermore, the results of a non-compartmental analysis of the changes in plasma antibody concentration are shown in Table 4. The clearance was 8.23 mL/d/kg with LBA and 5.47 mL/d/kg with ICP-MS, which is comparable.

These results indicate that the pharmacokinetics of an iodine-labeled agent can be evaluated by the Method V of the present invention.

**[Table 4]**

| NCA Analysis Parameters in Mouse PK Study | | | | | |
|---|---|---|---|---|---|
| | | LBA | | ICP-MS | |
| | | Mean | S.D. | Mean | S.D. |
| Vss | mL/kg | 76.0 | 13.6 | 87.1 | 13.0 |
| Clearance | mL/d/kg | 8.23 | 0.11 | 5.47 | 1.21 |

### [Example 4] Cassette-dosing PK of indium-DOTA-labeled and iodine-labeled denosumab in WT mice

### 1. Labeling

Preparation of indium-DOTA-labeled denosumab: A Zeba spin desalting column (40 K, 2 mL, Thermo Fisher Scientific) was used to replace the solvent of denosumab (Ranmark subcutaneous injection, Daiichi Sankyo) with PBS. 0.58 µmol of In-p-SCN-Bn-DOTA dissolved in DMSO was added to 11.6 nmol of denosumab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).

Preparation of iodine-labeled denosumab: A Zeba spin desalting column (40 K, 2 mL, Thermo Fisher Scientific) was used to replace the solvent of denosumab (Ranmark subcutaneous injection, Daiichi Sankyo) with PBS. To an iodination tube (manufactured by Pierce), 0.1 µmol of sodium iodide dissolved in 1 mM NaOH solution and Tris buffer were added, then the mixture was stirred and allowed to react at room temperature for 6 minutes. The obtained reaction solution was added to 20 nmol of denosumab solution, and the mixture was stirred and allowed to react at room temperature for 6 min. The obtained reaction solution was purified using PD-10 (manufactured by Cytiva). PBS-T was used as the eluent.

### 2. Calculation of labeling efficiency using ICP-MS

Nitric acid was added to the indium-labeled antibody sample, which was then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The obtained measured value was compared with the calibration curve to calculate the concentration of indium in the solution. The indium concentration in the labeled antibody solution of known concentration was measured, and the labeling efficiency was calculated from the result. The labeling efficiency was 48%.

After adding sodium hypochlorite to the iodine-labeled antibody sample, iodine in the solution was measured by ICP mass spectrometry. An iodine standard solution was also prepared and subjected to ICP-MS analysis, and a calibration curve was created based on the data obtained. The obtained measured value was compared with the calibration curve to calculate the concentration of iodine in the solution. The iodine concentration in the labeled antibody solution of known concentration was measured, and the labeling efficiency was calculated from the result. The labeling efficiency was 542%.

### 3. Animal administration and blood collection

The antibodies labeled in 1 above were simultaneously administered intravenously to C57BL6J mice as a single dose of 1 mg/kg for indium-DOTA-labeled denosumab and 10 mg/kg for iodine-labeled denosumab, for a total of 11 mg/kg as antibodies, through the tail vein. Blood samples were collected a plurality of times over time, from 5 minutes to 7 days after administration. Whole blood was collected from the abdominal vena cava under isoflurane anesthesia at 7 days after administration, followed by harvest of tissues (liver, lung, kidney, heart, spleen, and thigh muscle). Plasma was separated by centrifuging a portion of the obtained blood. All organs, blood and plasma were stored in a freezer set to -20°C or less until measurement.

### 4. Evaluation of antibody concentration in mouse blood, plasma and tissue using ICP-MS

### (1) Measurement of iodine concentration in mouse blood, plasma and tissue and calculation of antibody concentration using ICP-MS

After adding sodium hypochlorite to the mouse blood and plasma samples, and making it to constant volume with ultrapure water, iodine in the solution was measured by ICP mass spectrometry. An iodine standard solution was also prepared and subjected to ICP-MS analysis, and a calibration curve was created based on the data obtained. The obtained measured value was compared with the calibration curve to calculate the concentration of iodine in the solution. From the labeling efficiency and iodine concentration in the mouse blood and plasma samples, the antibody concentration in the samples was calculated.

Tetramethylammonium hydroxide (TMAH) was added to mouse tissue (liver, lung, kidney, heart, spleen, and thigh muscle) samples, which were kept in a thermostatic bath at 60°C for 3 hours to decompose. A solution made to constant volume with ultrapure water was used as the sample solution and measured with an ICP mass spectrometer. An iodine standard solution was also prepared and subjected to ICP-MS analysis, and a calibration curve was created based on the data obtained. The obtained measured value was compared with the calibration curve to calculate the concentration of iodine in the solution. From the labeling efficiency and iodine concentration, the antibody concentration in the mouse tissue samples was calculated.

### (2) Measurement of indium concentration in mouse blood, plasma and tissue and calculation of antibody concentration using ICP-MS

Nitric acid was added to the mouse blood, plasma, and tissue (liver, lung, kidney, heart, spleen, and thigh muscle) samples, which were then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The measured value of the sample solution was compared with the calibration curve to calculate the concentration of indium in the solution. It is also possible to add a rhodium solution as the internal standard solution to each sample and standard solution and calculate the indium concentration in each sample by the method of internal standards. From the labeling efficiency and indium concentration, the antibody concentration in the mouse blood and plasma samples was calculated.

### (3) Results

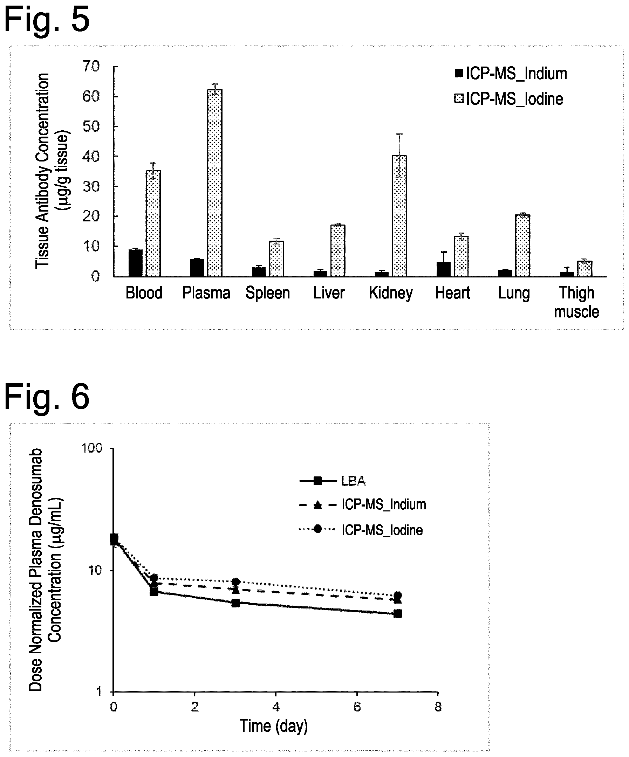

The results of measuring the antibody concentrations in organs 7 days after administration are shown in Figure 5. Iodine and indium were detected not only in blood and plasma, but also in each tissue (liver, lung, kidney, heart, spleen, and thigh muscle), and the tissue antibody concentration could be calculated. This suggests that the antibody concentration in the organs of mice given iodine-labeled denosumab and indium-DOTA-labeled denosumab can be evaluated by ICP-MS.

### 5. Comparison of the measurement results using ICP-MS with the measurement results using LBA

### (1) Measurement of antibody concentration in mouse plasma using LBA

The mouse plasma samples were diluted 100-fold with 1% BSA in PBS-T, then further diluted 30- or 900-fold with 1% plasma/1% BSA in PBS-T (final: 3000 - 90000-fold dilution), and used as the plasma sample for measurement. To create the calibration curve, a 10:1 mixed solution of iodine-labeled denosumab and indium-DOTA-labeled denosumab was diluted with 1% plasma/1% BSA in PBS-T, and diluted solutions of 50, 20, 10, 5, 2, 1, and 0.5 ng/mL were prepared. Anti-human IgG antibodies (Bethyl) were added to sector plates (Meso scale discovery) as the solid phase antibody. Biotin-labeled anti-human antibodies (SouthernBiotech) were added as the detection antibody, and after incubation, Sulfo-tag-labeled streptavidin (Meso scale discovery) was added. After incubation, Read buffer (Meso scale discovery) was added and the content of the test substance was measured to calculate the concentration in plasma.

### (2) Results

A graph plotting the plasma antibody concentrations measured by ICP-MS and LBA in C57BL6J mice given iodine-labeled denosumab and indium-DOTA-labeled denosumab, expressed as dose-normalized values at 1 mg/kg, is shown in Figure 6.

In C57BL6J mice, the changes in plasma concentration of iodine-labeled denosumab and indium-DOTA-labeled denosumab measured by ICP-MS were comparable to the changes in plasma antibody concentration measured by LBA.

Furthermore, the results of a non-compartmental analysis of the changes in plasma antibody concentration are shown in Table 5. The clearance was 9.0 mL/d/kg for LBA, 5.9 mL/d/kg for the measurement of iodine concentration by ICP-MS, and 6.1 mL/d/kg for the measurement of indium concentration by ICP-MS. This indicates that measuring the plasma iodine and indium concentrations in mice given iodine-labeled denosumab and indium-DOTA-labeled denosumab by ICP-MS allowed to simultaneously evaluate the changes in plasma concentration of antibodies labeled with different elements. The results of the present example suggest that it is possible to administer two or more agents labeled with different non-radioactive substances to a single animal and simultaneously evaluate the changes in plasma concentration of these agents.

**[Table 5]**

| NCA Parameters for Changes in Plasma Antibody Concentration in Mouse PK Study | | | | | | | |
|---|---|---|---|---|---|---|---|
| | | LBA | | ICP-MS_Indium | | ICP-MS_Iodine | |
| | | Mean | S.D. | Mean | S.D. | Mean | S.D. |
| Vss | mL/kg | 133 | 15.4 | 115 | 11.3 | 102 | 2.2 |
| Clearance | mL/d/kg | 9.0 | 1.90 | 6.1 | 0.43 | 5.9 | 0.39 |

### [Example 5] PK of cetuximab labeled with four different metals (In, Tb, Y, and Ho) in wild-type mice (In, Tb, Y, Ho-labeled-cetuximab PK in WT mice)

### 1. Labeling reagent

Tb-p-SCN-Bn-DOTA and Y-p-SCN-Bn-DOTA were obtained by contract synthesis from Macrocyclics, Inc. They have a structure in which In in In-p-SCN-Bn-DOTA is replaced by Tb and Y, respectively.

Ho-p-SCN-Bn-DOTA was obtained from Macrocyclics, Inc.

### 2. Labeling

(1) Y-labeled Cetuximab: A Zeba spin desalting column (40 K, 2 mL, Thermo Fisher Scientific) was used to replace the solvent of cetuximab (Erbitux injection, Merck Biopharma) with PBS. 0.73 µmol of Y-p-SCN-Bn-DOTA dissolved in DMSO was added to 14.6 nmol of cetuximab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).
(2) Tb-labeled Cetuximab: A Zeba spin desalting column (40 K, 2 mL, Thermo Fisher Scientific) was used to replace the solvent of cetuximab (Erbitux injection, Merck Biopharma) with PBS. 0.75 µmol of Tb-p-SCN-Bn-DOTA dissolved in DMSO was added to 15.1 nmol of cetuximab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).
(3) In-labeled Cetuximab: A Zeba spin desalting column (40 K, 2 mL, Thermo Fisher Scientific) was used to replace the solvent of cetuximab (Erbitux injection, Merck Biopharma) with PBS. 6.2 µmol of In-p-SCN-Bn-DOTA dissolved in DMSO was added to 0.12 µmol of cetuximab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).
(4) Ho-labeled Cetuximab: A Zeba spin desalting column (40 K, 2 mL, Thermo Fisher Scientific) was used to replace the solvent of cetuximab (Erbitux injection, Merck Biopharma) with PBS. 0.73 µmol of Ho-p-SCN-Bn-DOTA (Microcyclics, Inc.) dissolved in DMSO was added to 14.6 nmol of cetuximab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).

### 3. Calculation of labeling efficiency using ICP-MS

Nitric acid was added to the labeled antibody samples, which were then decomposed in a microwave sample pretreatment device. Solutions made to constant volume with ultrapure water were used as the sample solutions and measured with an ICP mass spectrometer. Each standard solution of indium, terbium, yttrium, and holmium was also prepared and subjected to ICP-MS analysis, and calibration curves were created based on the data obtained. The obtained measured values were compared with the calibration curves to calculate the concentrations of metallic element in the solutions. The metallic element concentration in the labeled antibody solution of known concentration was measured, and the labeling efficiency was calculated from the result. The value (%) calculated from metallic element concentration (mol/L) / antibody concentration (mol/L) x 100 was used as the labeling efficiency. The labeling efficiencies for each metal labeling are shown in Table 6.

**[Table 6]**

| | Labeling Metal | Labeling Efficiency |
|---|---|---|
| 1 | Indium | 53% |
| 2 | Terbium | 143% |
| 3 | Yttrium | 153% |
| 4 | Holmium | 47% |

4. Animal administration 1 mg/kg each of indium-DOTA, yttrium-DOTA and holmium-DOTA-labeled antibodies labeled in 2 above and 0.94 mg/kg of terbium-DOTA-labeled antibodies, for a total of 3.94 mg/kg of antibodies, were simultaneously administered intravenously to C57BL6J mice as a single dose through the tail vein. Blood samples were collected a plurality of times over time, after 5 minutes to 7 days. Whole blood was collected from the abdominal vena cava under isoflurane anesthesia at 7 days after administration, followed by harvest of tissues (liver, lung, kidney, heart, spleen, and thigh muscle). Plasma was separated by centrifuging a portion of the obtained blood. All organs, blood and plasma were stored in a freezer set to -20°C or less until measurement.

### 5. Evaluation of antibody concentration in mouse blood, plasma and tissue using ICP-MS

### (1) Measurement of metal concentration in mouse blood, plasma and tissue and calculation of antibody concentration using ICP-MS

Nitric acid was added to the mouse blood, plasma, and tissue (liver, lung, kidney, heart, spleen, and thigh muscle) samples, which were then decomposed in a microwave sample pretreatment device. Solutions made to constant volume with ultrapure water were used as the sample solutions and measured with an ICP mass spectrometer. Each standard solution of indium, terbium, yttrium, and holmium was prepared and subjected to ICP-MS analysis, and calibration curves were created based on the data obtained. It is also possible to add a rhodium solution as the internal standard solution to each sample and standard solution and calculate the indium, terbium, yttrium and holmium concentrations in each sample by the method of internal standards. The obtained measured values were compared with the calibration curves to calculate the concentrations of metallic element in the solutions. From the labeling efficiency and each metal concentration, the antibody concentration in the mouse blood, plasma and tissue samples was calculated.

### (2) Results

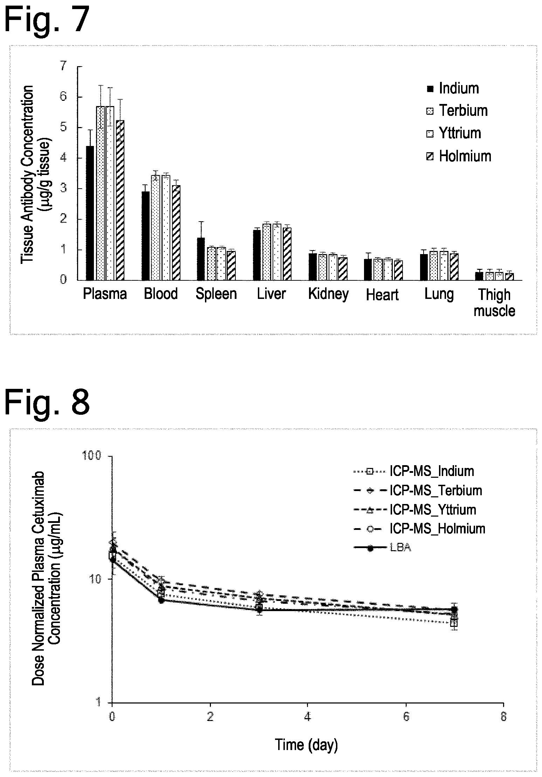

The results of measuring the antibody concentrations in organs 7 days after administration are shown in Figure 7. Indium, terbium, yttrium and holmium were detected not only in blood and plasma, but also in each tissue (liver, lung, kidney, heart, spleen, and thigh muscle), and the tissue antibody concentration could be calculated. This suggests that the antibody concentration in the organs of mice given indium-DOTA, terbium-DOTA, yttrium-DOTA and holmium-DOTA-labeled cetuximab can be evaluated by ICP-MS.

### 6. Comparison of the measurement results using ICP-MS with the measurement results using LBA

### (1) Measurement of antibody concentration in mouse plasma using LBA

The mouse plasma samples were diluted 100-fold with 1% BSA in PBS-T, then further diluted 30-fold with 1% plasma/1% BSA in PBS-T (final: 3000-fold dilution), and used as the plasma sample for measurement. To create the calibration curve, a 1:0.94: 1: 1 mixed solution of indium-DOTA-labeled antibodies, terbium-DOTA-labeled antibodies, yttrium-DOTA-labeled antibodies and holmium-DOTA-labeled antibodies was diluted with 1% plasma/1% BSA in PBS-T, and diluted solutions of 49.3, 19.7, 9.85, 4.93, 1.97, 0.985, and 0.493 ng/mL were prepared. Anti-human IgG antibodies (Bethyl) were added to sector plates (Meso scale discovery) as the solid phase antibody. Biotin-labeled anti-human antibodies (SouthernBiotech) were added as the detection antibody, and after incubation, Sulfo-tag-labeled streptavidin (Meso scale discovery) was added. After incubation, Read buffer (Meso scale discovery) was added and the content of the test substance was measured to calculate the concentration in plasma.

### (2) Results

A graph plotting the plasma antibody concentrations measured by ICP-MS and LBA in C57BL6J mice given indium-DOTA, terbium-DOTA, yttrium-DOTA and holmium-DOTA-labeled cetuximab, each expressed as dose-normalized values at 1 mg/kg, is shown in Figure 8.

In C57BL6J mice, the changes in plasma concentration of each metallic element-labeled antibody measured by ICP-MS were comparable to the changes in plasma concentration of total human IgG measured by LBA.

Furthermore, the results of a non-compartmental analysis of the changes in plasma antibody concentration are shown in Table 7. The clearance was 8.00 mL/d/kg for LBA, 10.7 mL/d/kg for indium-DOTA-labeled antibodies, 7.68 mL/d/kg for terbium-DOTA-labeled antibodies, 8.99 mL/d/kg for yttrium-DOTA-labeled antibodies, and 8.45 mL/d/kg for holmium-DOTA-labeled antibodies by ICP-MS. This indicates that measuring the plasma indium, terbium, yttrium and holmium concentrations in mice given indium-DOTA-labeled antibodies, terbium-DOTA-labeled antibodies, yttrium-DOTA-labeled antibodies and holmium-DOTA-labeled antibodies by ICP-MS allows to separately quantify the changes in plasma concentration of each antibody.

**[Table 7]**

| NCA Parameters for Changes in Plasma Antibody Concentration in Mouse PK Study | | | | | | | | | | | |
|---|---|---|---|---|---|---|---|---|---|---|---|
| | | LBA | | **ICP-MS_lndium** | | **ICP-MS_Terbium** | | **ICP-MS_Yttrium** | | **ICP-MS_Holmium** | |
| | | **Mean** | S.D. | **Mean** | S.D. | **Mean** | S.D. | **Mean** | S.D. | **Mean** | S.D. |
| Vss | mL/kg | 119 | 22.0 | 117 | 6.9 | 86.5 | 6.43 | 100 | 8.83 | 107 | 4.46 |
| Clea rance | mL/ d/kg | 8.00 | 3.98 | 10.7 | 1.58 | 7.68 | 1.27 | 8.99 | 1.26 | 8.45 | 1.49 |

### [Example 6] Indium-DOTA-labeled cetuximab PK in cynomolgus monkey (Indium-labeled cetuximab PK in cynomolgus monkey)

### 1. Labeling

In-labeled Cetuximab: A Zeba spin desalting column (40 K, 2 mL, Thermo Fisher Scientific) was used to replace the solvent of cetuximab (Erbitux injection, Merck Biopharma) with PBS. 5.8 µmol of In-p-SCN-Bn-DOTA dissolved in DMSO was added to 0.12 µmol of cetuximab solution, and the mixture was stirred at room temperature for 4 hours. A simple purification of the obtained reaction solution was performed using a Zeba spin desalting column, followed by purification by gel filtration using Superdex 200 increase 10/300 GL (Cytiva) as a column on an AKTA explorer 10S (Cytiva).

### 2. Calculation of labeling efficiency using ICP-MS

Nitric acid was added to the labeled antibody sample, which was then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The obtained measured value was compared with the calibration curve to calculate the concentration of indium in the solution. The indium concentration in the labeled antibody solution of known concentration was measured, and the labeling efficiency was calculated from the result. The labeling efficiency was 39%.

### 3. Animal administration and blood collection

1.5 mg/kg and 3.5 mg/kg of antibodies labeled by the method described in 1 above and unlabeled antibodies, respectively, for a total of 5 mg/kg of antibodies, were simultaneously administered intravenously to cynomolgus monkeys as a single dose through the cephalic vein. Blood samples were collected a plurality of times over time, from 5 minutes to 7 days after administration. At 7 days after administration, the monkeys were euthanized by exsanguination under anesthesia with a combination of ketamine hydrochloride and medetomidine hydrochloride, and then their tissues (liver, lung, kidney, heart, spleen, and thigh muscle) were harvested. Plasma was separated by centrifuging a portion of the obtained blood. All organs, blood and plasma were stored in a freezer set to -70°C or less until measurement.

### 4. Evaluation of antibody concentration in monkey blood, plasma and tissue using ICP-MS

### (1) Measurement of indium concentration in monkey blood, plasma and tissue and calculation of antibody concentration using ICP-MS

Nitric acid was added to the monkey blood, plasma, and tissue (liver, lung, kidney, heart, spleen, and thigh muscle) samples, which were then decomposed in a microwave sample pretreatment device. A solution made to constant volume with ultrapure water was used as the sample solution and measured with an ICP mass spectrometer. An indium standard solution was prepared and subjected to ICP-MS analysis, and a calibration curve was created based on the data obtained. It is also possible to add a rhodium solution as the internal standard solution to each sample and standard solution and calculate the indium concentration in each sample by the method of internal standards. The obtained measured value was compared with the calibration curve to calculate the concentration of indium in the solution. From the labeling efficiency and indium concentration, the antibody concentration in the monkey blood, plasma and tissue samples was calculated.

### (2) Results

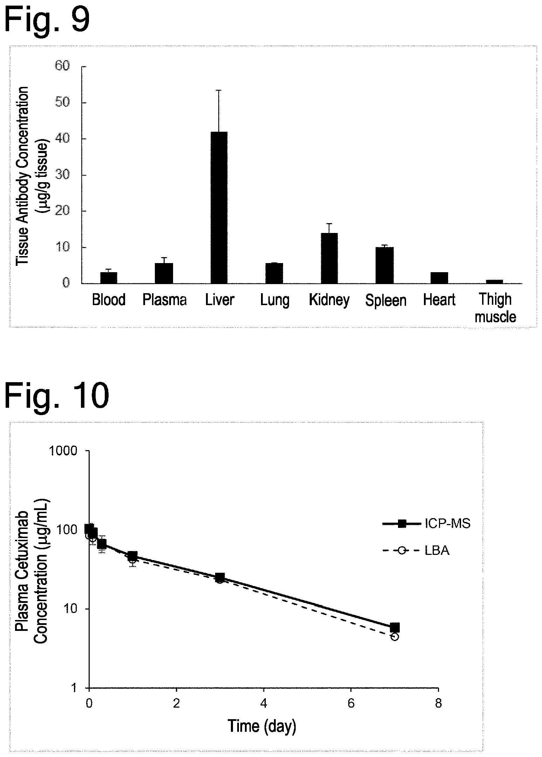

The results of measuring the antibody concentrations in organs 7 days after administration are shown in Figure 9. Indium was detected not only in blood and plasma, but also in each tissue (liver, lung, kidney, heart, spleen, and thigh muscle), and the tissue antibody concentration could be calculated. This suggests that the antibody concentration in the organs of monkeys given indium-DOTA-labeled cetuximab can be evaluated by ICP-MS.

### 5. Comparison of the measurement results using ICP-MS with the measurement results using LBA

### (1) Measurement of antibody concentration in monkey plasma using LBA

The monkey plasma samples were diluted 100-fold with 1% BSA in PBS-T, then further diluted 20-200-fold with 1% plasma/1% BSA in PBS-T (final: 2000-20000-fold dilution), and used as the plasma sample for measurement. To create the calibration curve, unlabeled cetuximab was diluted with 1% BSA in PBS-T, and diluted solutions of 50, 20, 10, 5, 2, 1, and 0.5 ng/mL were prepared. Human EGFR (Sino Biological) was added to sector plates (Meso scale discovery) as the solid phase antigen. After adding sTag-labeled anti-human antibodies (Meso scale discovery) as the detection antibody and incubating, Read buffer (Meso scale discovery) was added and the content of the test substance was measured to calculate the concentration in plasma.

### (2) Results

The changes in plasma antibody concentration measured by ICP-MS and LBA in cynomolgus monkeys given indium-DOTA-labeled cetuximab are shown in Figure 10.

In cynomolgus monkeys, the changes in plasma concentration of indium-DOTA-labeled cetuximab measured by ICP-MS were comparable to the changes in plasma antibody concentration measured by LBA.

Furthermore, the results of a non-compartmental analysis of the changes in plasma antibody concentration are shown in Table 8. The clearance was 28.0 mL/d/kg with LBA and 24.8 mL/d/kg with ICP-MS. This suggests that the changes in plasma concentration of cetuximab can be evaluated by measuring the plasma concentration of indium in cynomolgus monkeys given indium-DOTA-labeled cetuximab by ICP-MS.

**[Table 8]**

| NCA Parameters for Changes in Plasma Antibody Concentration in Monkey PK Study | | | | | |
|---|---|---|---|---|---|
| | | LBA | | ICP-MS | |
| | | Mean | S.D. | Mean | S.D. |
| Vss | mL/kg | 72.5 | 16.1 | 69.8 | 12.6 |
| Clearance | mL/day/kg | 28.0 | 3.43 | 24.8 | 1.15 |

### [Example 7] Application examples in molecular forms of non-antibodies

### (Application examples in non-antibody modalities)

1. Medium-sized molecule (peptide) labeling 15 µmol of Bacitracin (Fujifilm Wako) were dissolved in DMSO, 18 µmol of In-p-SCN-Bn-DOTA dissolved in DMSO and 200 µL of DMA were added and the mixture was stirred. 76 µmol of diisopropylethylamine was added and the mixture was stirred at room temperature for 30 minutes. The reaction solution was purified in an automated purification preparative system Isolera One (Biotage) using Biotage (registered trademark) Sfar C18 (Biotage), and the solvent was removed by lyophilization.

### 2. Calculation of labeling efficiency of medium-sized molecule using ICP-MS

Nitric acid was added to the labeled peptide sample, which was then decomposed in a microwave sample pretreatment device. A solution made to constant volume with ultrapure water was used as the sample solution and measured with an ICP mass spectrometer. An indium standard solution was also prepared and subjected to ICP-MS analysis, and a calibration curve was created based on the data obtained. The obtained measured value was compared with the calibration curve to calculate the concentration of indium in the solution. The indium concentration in the labeled peptide solution of known concentration was measured, and the labeling efficiency was calculated from the result. The labeling efficiency was 46%.

Indium-DOTA-labeled Bacitracin prepared in this way can be used to evaluate the PK of Bacitracin as in the Examples described above.

### INDUSTRIAL APPLICABILITY

According to the present invention, it is possible to measure the pharmacokinetics of drug development candidates in laboratory animals without using RI, and to conduct pharmacokinetic studies from the early stages of development. The present invention can also contribute to the reduction of the number of laboratory animals used and to the development of more pharmacologically effective drugs.

## Claims

1. A method for labeling an agent, the method comprising:
(i) a step of chelating a non-radioactive substance by a chelating agent having a reactive group, and
(ii) a step of binding the chelating agent that chelated the non-radioactive substance in step (i) to the agent via the reactive group,
the method being for use in the evaluation of pharmacokinetics.

2. The method according to claim 1, wherein the non-radioactive substance is a metal.

3. The method according to claim 2, wherein the non-radioactive substance is lithium, beryllium, sodium, magnesium, aluminum, potassium, calcium, scandium, titanium, vanadium, chromium, manganese, iron, cobalt, nickel, copper, zinc, gallium, rubidium, strontium, yttrium, zirconium, niobium, molybdenum, ruthenium, rhodium, palladium, silver, cadmium, indium, tin, cesium, barium, lanthanum, cerium, praseodymium, neodymium, samarium, europium, gadolinium, terbium, dysprosium, holmium, erbium, thulium, ytterbium, lutetium, hafnium, tantalum, tungsten, rhenium, osmium, iridium, platinum, gold, mercury, thallium, lead, bismuth, or thorium.

4. The method according to any one of claims 1 to 3, wherein the chelating agent having a reactive group is a chelating agent having an amino group or a carboxyl group as a coordinating group.

5. The method according to claim 4, wherein the chelating agent having a reactive group is a chelating agent having a reactive group introduced into any compound selected from DOTA, EDTA, HEDTA, DHEDDA, 1,3-PDTA, DTPA, TTHA, NTA, gluconic acid, HIMDA, ASDA, NTMP, HEDP, tetrasodium 3-hydroxy-2,2'-iminodisuccinate, and porphyrin.

6. The method according to any one of claims 1 to 5, wherein the agent is an antibody, a peptide compound, a cell, or a nucleic acid.

7. The method according to any one of claims 1 to 6, wherein the reactive group is a group capable of forming a chemical bond by reacting with an amino group or a thiol group.

8. A method for measuring the pharmacokinetics of an agent, the method comprising:
(i) a step of labeling the agent with a non-radioactive substance by the method according to any one of claims 1 to 7,
(ii) a step of administering the agent labeled in step (i) to a non-human animal, and
(iii) a step of collecting a biological sample from the non-human animal after administration of the agent in step (ii) and measuring the content of the non-radioactive substance in the biological sample.

9. The method according to claim 8, wherein in step (i), two or more agents are labeled with different non-radioactive substances,
and in step (ii), the two or more agents labeled in step (i) are administered to a single non-human animal.

10. The method according to claim 8 or 9, wherein the content of the non-radioactive substance is measured by mass spectrometry.

11. The method according to claim 10, wherein the mass spectrometry is plasma ionization mass spectrometry, electron ionization (EI) mass spectrometry, chemical ionization (CI) mass spectrometry, atmospheric pressure chemical ionization (APCI) mass spectrometry, electrospray ionization (ESI) mass spectrometry, matrix-assisted laser desorption/ionization (MALDI) mass spectrometry, laser desorption mass spectrometry (LDMS), secondary ion mass spectrometry (SIMS), spark source mass spectrometry (SSMS), or thermal ionization mass spectrometry (TIMS).

12. The method according to claim 11, wherein the mass spectrometry is plasma ionization mass spectrometry.

13. The method according to any one of claims 8 to 12, wherein the non-human animal is a monkey, miniature pig, rat, mouse, rabbit, dog, or guinea pig.

14. The method according to any one of claims 8 to 13, wherein the administration of the agent in step (ii) is intravenous administration (i.v.), subcutaneous administration (s.c.), peroral administration (p.o.), intraperitoneal administration (i.p.), intramuscular administration (i.m.), intratumoral administration (i.t.), transpulmonary administration, or intranasal administration.

15. A method for screening agents having a desired pharmacokinetics, the method comprising:
(i) a step of measuring the pharmacokinetics of two or more candidate agents by the method according to any one of claims 8 to 14, and
(ii) a step of comparing the pharmacokinetics of the candidate agents measured in step (i) and selecting an agent exhibiting the desired pharmacokinetics.
